# EUROPÄISCHE PATENTSCHRIFT

(11) **EP 2 544 812 B1**
(45) Veröffentlichungstag und Bekanntmachung des Hinweises auf die Patenterteilung: **24.10.2018**
(21) Anmeldenummer: 11714683.7
(22) Anmeldetag: 14.03.2011
(51) Int. Cl.: B01J 13/14, B01J 13/20

(54) **VERBESSERTE MIKROKAPSELN UND IHRE HERSTELLUNG**
IMPROVED MICROCAPSULES AND PRODUCTION THEREOF
MICROCAPSULES AMÉLIORÉES ET LEUR PRÉPARATION

(30) Priorität: 13.09.2010 EP 10009497; 12.03.2010 WO PCT/EP2010/001572
(43) Veröffentlichungstag der Anmeldung: 16.01.2013
(73) Patentinhaber: Follmann GmbH & Co. KG, 32423 Minden (DE)
(72) Erfinder: LAST, Klaus, 37520 Osterode (DE); MUES, Daniel, 31633 Leese (DE)
(74) Vertreter: von Renesse, Dorothea
(86) Internationale Anmeldenummer: PCT/EP2011/001253
(87) Internationale Veröffentlichungsnummer: WO 2011/110368

(56) Entgegenhaltungen:
- EP-A1- 0 120 504
- EP-A2- 0 152 083
- WO-A2-2010/102830
- US-A- 4 936 916
- US-A- 5 422 176

## Beschreibung

Die Erfindung betrifft Mikrokapseln, deren Kapselwände ein Harz umfassen, welches erhältlich ist durch Umsetzung mindestens eines Amins und/oder bestimmter Aromaten oder Heteroaromaten und mindestens einer aldehydischen Komponente, die mindestens zwei C-Atome pro Molekül aufweist, in Gegenwart mindestens eines Copolymeren, welches Einheiten von AMPS und/oder AMPP und (Meth)Acrylaten enthält. Darüberhinaus ist Gegenstand der Erfindung die Herstellung der Mikrokapseln.

Aus dem Stand der Technik sind Mikrokapseln bekannt, die als Kernmaterial flüssige, feste oder gasförmige Stoffe enthalten können. Als Material für die Kapselwände sind beispielsweise Phenol-Formaldehyd-Polymere, Melamin-Formaldehyd-Polymere, Polyurethan, Gelatine, Polyamide oder Polyharnstoffe gebräuchlich. Weit verbreitet ist beispielsweise die Verwendung Leukofarbstoff-gefüllter Mikrokapseln zur Herstellung selbstdurchschreibender Papiere.
Aus US 3,755,190 ist bekannt, dass Kapseln aus Phenol-Formaldehyd-Polymeren brüchige Wände aufweisen. Um dies zu vermeiden, wird ein Herstellverfahren beschrieben, bei welchem vollständig hydrolysierter Polyvinylalkohol eingesetzt wird. Dispersionen von Mikrokapseln aus Aminoplastharzen, wie Melamin-Formaldehyd-Harzen, enthalten herstellungsbedingt einen gewissen Anteil freien Formaldehyds. Es ist aus umwelt- und arbeitshygienischen Gründen anzustreben, den Formaldehydgehalt so niedrig wie möglich zu halten, wenn möglich Formaldehyd ganz zu vermeiden. Zur Verringerung des Formaldehyd-Gehalts ist es üblich, Mikrokapseldispersionen auf der Basis von Melamin-Formaldehyd-Harzen Formaldehydfänger zuzusetzen. Zu den am häufigsten verwendeten Formaldehydfängern gehören Ammoniak, Harnstoff, Ethylenharnstoff und Melamin, die den Restgehalt an Formaldehyd in der Kapseldispersion reduzieren.

Aus EP-A 0 383 358 und DE-A 38 14 250 sind lichtempfindliche Materialien bekannt, die aus Mikrokapseln bestehen, deren Wände aus Melamin-Formaldehyd-Harzen gebildet werden. Zur Entfernung überschüssigen Formaldehyds wird bei der Härtung Harnstoff zugesetzt.

Bei den in der EP-A 319 337 und US 4,918,317 beschriebenen Verfahren wird Harnstoff gegen Ende der Härtung zugesetzt.

Die EP-A 0 415 273 beschreibt die Herstellung und Verwendung mono- und polydisperser Vollkugelteilchen aus Melamin-Formaldehyd-Kondensat. Zur Bindung des bei der Kondensation freiwerdenden Formaldehyds wird die Verwendung von Ammoniak, Harnstoff oder Ethylenharnstoff vorgeschlagen.

Mikrokapseln aus Melamin-Formaldehyd-Harzen, die unter Verwendung sulfonsäuregruppen-haltiger Polymere hergestellt werden, zeichnen sich durch ihre gleichmäßige Kapselgröße und Dichtigkeit aus (EP-A 0 218 887 und der EP-A 0 026 914.) Diese Kapseldispersionen enthalten jedoch noch restlichen freien Formaldehyd, dessen Anwesenheit bei der Weiterverarbeitung unerwünscht ist.

Die EP-A 0 026 914 empfiehlt daher, den Formaldehyd im Anschluss an die Härtung mit Ethylenharnstoff und/oder Melamin als Formaldehydfänger zu binden.

Aus der DE 198 35 114 sind Dispersionen von Mikrokapseln auf der Basis von Melamin-Formaldehyd-Harz bekannt, wobei das Melamin-Formaldehyd-Harz teilweise verethert ist und ein wasserlösliches primäres, sekundäres oder tertiäres Amin oder Ammoniak enthält. Vor der Härtung wird als Formaldehydfänger Harnstoff zugesetzt.

Die DE 198 33 347 beschreibt ein Verfahren zur Herstellung von Mikrokapseln durch Kondensation von Melamin-Formaldehyd-Harzen und/oder deren Methylethern, wobei vor der Härtung Harnstoff oder Harnstoff, dessen Aminogruppen mit einer Ethylen- oder Propylenbrücke verbunden sind, als Formaldehydfänger zugesetzt wird. Die erhaltenen Dispersionen sind zwar formaldehydarm, durch den Zusatz von Harnstoff vor der Härtung werden jedoch die Stabilität der Mikrokapseln und die Viskosität der Mikrokapseldispersion ungünstig beeinflusst.
Die WO 01/51197 lehrt ein Verfahren zur Herstellung von Mikrokapseln durch Kondensation von Melamin-Formaldehyd-Harzen, bei dem während der Härtung ein Gemisch aus Melamin und Harnstoff zugesetzt wird.

EP 0 120 504 A1 offenbart ein Verfahren zur Herstellung von Mikrokapseln, wobei Melamin-Formaldehyd Vorkondensat mit Furfural, Glutaraldehyd oder Glyoxal in Gegenwart eines 2-Acrylamido-2-Methyl-propansulfonsäure Copolymers und eines Kernmaterials zur Reaktion gebracht werden.
Durch den Zusatz der genannten Formaldehydfänger zur fertigen Mikrokapseldispersion bzw. bei der Herstellung der Mikrokapseldispersion wird regelmäßig der Formaldehydgehalt der Dispersion gesenkt. Oft lässt sich jedoch der Formaldehydgehalt von Produkten, die derartige Mikrokapseldispersionen enthalten oder damit behandelt wurden auch bei Zugabe grosser Mengen an Formaldehydfänger nicht unterhalb eine bestimmte Grenze senken.
Aufgabe der vorliegenden Erfindung ist es deshalb, Mikrokapseln mit einem möglichst niedrigen Formaldehydgehalt zu entwickeln bzw. bevorzugt auf die Verwendung von Formaldehyd für Mikrokapseln ganz zu verzichten.

Diese Aufgaben wird gelöst durch die erfindungsgemäßen Mikrokapseln des Anspruchs 1.

Die Offenbarung betrifft außerdem Mikrokapseldispersionen enthaltend derartige erfindungsgemäße Mikrokapseln.
Überraschenderweise können erfindungsgemäß stabile Kern-Schale Mikrokapseln mit hoher chemischer und physikalischer Resistenz hergestellt werden, die den Anforderungen und Machbarkeit der industriellen Fertigung (Scale up) genügen. Dabei können in-situ aus den Bausteinen a) und b) Vorkondensate hergestellt werden, welche auch in einem Eintopfverfahren direkt weiter zu Öl in Wasser oder Wasser in Öl Mikroverkapselungen verwendet werden können.
Desweiteren stellt die Erfindung ein Verfahren zur Herstellung erfindungsgemäßer Mikrokapseln Verfügung, nach Anspruch 17.

Die Herstellung derartiger Mikrokapseldispersionen gelingt über den Einsatz geeigneter Vorkondensate aber auch in situ im Eintopfverfahren.
In der Definition der Komponente a) sind die aromatischen oder heteroaromatischen Verbindungen a2) von Aminen verschieden, die Verbindungen a2) umfassen somit keine Amine a1). Als Komponente a) können eine oder mehrere (z.B. zwei, drei oder vier) Verbindungen, vorzugsweise eine Verbindung, aus den Gruppen a1) und/oder a2) eingesetzt werden. Es können z.B. ein oder mehrere (z.B. zwei, drei oder vier) Amine a1) oder ein oder mehrere (z.B. zwei, drei oder vier) aromatische oder heteroaromatische Verbindungen a2). Als Verbindungen a2) sind aromatische oder heteroaromatische Alkohole (oder deren Ether und Ester) und/oder ein oder mehrere (z.B. zwei, drei oder vier) aromatische oder heteroaromatische Carbonsäuren (oder deren Ester) bevorzugt, besonders bevorzugte Verbindungen a2) sind aromatische Alkohole (oder deren Ether und Ester).
Wenn mehrere (z.B. zwei, drei oder vier) Verbindungen a) eingesetzt werden, können zwei oder mehr Verbindungen a1), zwei oder mehr Verbindungen a2), oder zwei oder mehr Verbindungen aus verschiedenen Untergruppen a1) oder a2) eingesetzt werden, z.B. ein oder mehrere (z.B. zwei) Verbindungen a1), ein oder mehrere (z.B. zwei) Verbindungen a2), oder ein oder mehrere (z.B. zwei) Verbindungen a1) und ein oder mehrere (z.B. zwei) Verbindungen a2), beispielsweise ein Amin und ein aromatischer Alkohol wie Harnstoff-Resorcin, Harnstoff-Phloroglucin, Melamin-Resorcin oder, Melamin-Phloroglucin. Damit eröffnet die vorliegende Erfindung eine Vielzahl an Wandmaterialien auf Duroplastbasis, welche formaldehydfrei sind und speziell auf die jeweiligen Anforderungsprofile der industriellen Anwendung zugeschnitten werden können.

Bevorzugt wird als Komponente a) nur eine Verbindung eingesetzt, vorzugsweise ein Amin a1) oder eine aromatische oder heteroaromatische Verbindung a2) wie ein aromatischer Alkohol.

Als Komponente a) besonders bevorzugt sind die Amine a1), gegebenenfalls in Kombination mit Verbindungen a2).

Als Amine a1) werden im Rahmen der vorliegenden Erfindung beispielsweise ayclische, aromatische oder heteroaromatische, vorzugsweise acyclische oder heteroaromatische Amine eingesetzt. Die Amine können eine oder mehrere Amingruppen aufweisen. Vorzugsweise weisen die Amine mehrere Amingruppen auf, insbesondere zwei oder drei Amingruppen. Weiter bevorzugt sind Amine mit mindestens einer Aminfunktion, die mindestens ein Wasserstoffatom aufweist. Bevorzugt sind primäre und sekundäre Amine. Besonders bevorzugt sind Amine mit zwei oder drei Aminofunktionen, die primäre oder sekundäre Aminfunktionen darstellen. Ganz besonders bevorzugt sind Amine, die zwei oder drei primäre Aminofunktionen aufweisen. Weiter bevorzugt sind Amine, die Schiffsche Basen, Imine oder Enamine in der Reaktion mit Aldehyden b) bilden.

Erfindungsgemäß kommen als Amine a1) z.B. folgende Verbindungen in Frage:
C₁-C₂₀-Alkylamine wie 1,2-Diaminohexan, 1,3-Diaminohexan, 1,2-Diaminodecan, 1,3,5-Triaminoeicosan,
Harnstoffe wie Harnstoff, Methylharnstoff, Dimethylharnstoff, Methylolharnstoffe, die teilweise oder vollständig verethert oder verestert sein können wie Methylolharnstoff, Dimethylolharnstoff und Di(methylmethylol)harnstoff,
Thioharnstoffe wie Thioharnstoff, Methylthioharnstoff, Dimethylthioharnstoff, Methylolthioharnstoffe, die teilweise oder vollständig verethert oder verestert sein können wie Methylolthioharnstoff, Dimethylolthioharnstoff und Di(methylmethylol)thioharnstoff,
Triazine wie Melamine, z.B. Melamin, Methylolmelamine, die teilweise oder vollständig verethert oder verestert sein können wie Hexamethylolmelamin oder methyliertes Hexamethylolmelamin,
Imino-Melamine, z.B. Iminomelamin,
Guanidine, z.B. Guanidin; Benzylguanidin und Guanidincarbonat,
Guanine, z.B. Guanin,
Uracile, z.B. Uracil,
Thymine, z.B. Thymin,
Cytosine, z.B. Cytosin,
Adenine, z.B. Adenin,
Benzoguanamine, z.B. Benzoguanamin, Acetoguanamin,
Benzotriazole, z.B. Benzotriazol,
Glycourile, z.B. Glycouril,
Indole, z.B. Indol und mit primären oder sekundären Amingruppen substituierte Indole, Pyrole, z.B. Pyrol und mit primären oder sekundären Amingruppen substituierte Pyrole, Pyridine, z.B. Pyridin und mit primären oder sekundären Amingruppen substituierte Pyridine,
Pyrimidine, z.B. Pyrimidin, und mit primären oder sekundären Amingruppen substituierte Pyrimidine wie alpha Aminopyrimidin,
Pyrazine, z.B. Pyrazin und mit primären oder sekundären Amingruppen substituierte Pyrazine,
Chinoline, z.B. Chinolin und mit primären oder sekundären Amingruppen substituierte Chinoline.

Die vorgenannten aromatischen oder heteroaromatischen Amine können am cyclischen Grundkörper neben Aminofunktionen zusätzlich z.B. folgende Substituenten noch tragen: C₁-C₂₀-Alkyl, OH, OR, SH, SR, COOH, NHCOR, OCOR, SO₃H, PO₃H, Halogen (F, Cl, Br, I), C₆-C₁₄-Aryl wie unsubstituiertes oder substituiertes Phenyl oder Naphthyl (z.B. substituiert durch C₁-C₁₀Alkyl, C₁-C₁₀-Alkoxy, Halogen, Halogen-C₁-C₁₀-Alkyl, oder Halogen-C₁-C₁₀-Alkoxy), wobei R eine C₁-C₂₀-Alkyl Gruppe darstellt und die Gruppen OH, SH, COOH, SO₃H, PO₃H auch in Form ihrer Salze vorliegen können.

Bei der Umsetzung von Aminkomponente und Aldehyden liegt das Molverhältnis zwischen am Stickstoff gebundenen Wasserstoffatomen und Aldehydfunktionen im Allgemeinen zwischen 0,01 und 1:1, vorzugsweise zwischen 0,2 und 1:1. Die Reaktionsprodukte bieten durch geeignete Wahl der Reaktivitäten der eingesetzten Komponenten die Möglichkeit, die für die Ausbildung der Kapselwand erforderliche Bildungsgeschwindigkeit des Wandmaterials, die Netzwerkdichte, die Wandstärke und die Art des duroplastischen Wandmaterials gezielt auf die Anforderungen abzustimmen.

Bevorzugte Komponenten a1) sind Harnstoffe, Melamine, und Benzoguanamine und deren Mischungen. Besonders bevorzugte Komponenten a1) sind Harnstoff, Melamin und Benzoguanamin und deren Mischungen, ganz besonders bevorzugt Harnstoff, Melamin und Harnstoff/Melamin.

Die Herstellung der Aminoharzkondensate erfolgt nach den für die Herstellung von Aminoplastkondensaten üblichen Methoden. Durch Umsetzung von Aldehyden b) mit a1) Komponenten entstehen Polykondensate, die OH-Gruppen in alpha-Stellung zu Amin-Gruppierungen aufweisen.

Die geeignete Reaktionstemperatur für die Herstellung der Aminoharzprodukte aus Amin a1) und Aldehyd liegt im Allgemeinen zwischen 20°C und 90°C, vorzugsweise zwischen 40°C und 60°C, bei pH-Werten im Allgemeinen zwischen 2 und 10.

Die Reaktion kann in wässrigen wie auch in organischer Phase durchgeführt werden. Geeignete Lösungsmittel sind Wasser, Alkohole, aromatische oder aliphatische Kohlenwasserstoffe wie z.B. Mineralöle, Myristate usw. Besonders bevorzugt ist die Reaktion in wässriger Phase.

Weiterhin sind als Komponente a) aromatische und heteroaromatische Verbindungen a2) geeignet. Diese können eine elektrophile Reaktion mit der Aldehydkomponente eingehen und anschließend Polykondensationsreaktionen erlauben.

Beispiele für aromatische und heteroaromatische Verbindungen a2) sind unsubstituierte aromatische und heteroaromatische Verbindungen wie Inden, Benzol, Toluol.

Bevorzugt geeignete Verbindungen a2) sind substituierte aromatische und heteroaromatische Verbindungen.

Dabei sind Aryloxyalkanole, Arylalkanole und Oligoalkanolarylether bevorzugt, geeignet sind auch Verbindungen, die elektronenreichen Doppelbindungen zur Verfügung stellen, z.B. Enolether oder Enaminsysteme wie Benzofuran, Furan, Pyran.

Besonders bevorzugte Verbindungen a2) sind aromatische Alkohole und deren Ether oder Derivate, wobei als Derivate Ester bevorzugt sind.

Ganz besonders bevorzugt sind aromatische und heteroaromatische, vorzugsweise aromatische Verbindungen, bei denen mindestens eine freie Hydroxygruppe oder Carbonsäuregruppe, besonders bevorzugt mindestens zwei freie Hydroxy- oder Carbonsäure-Gruppen unmittelbar am aromatischen oder heteroaromatischen Ring gebunden sind. Dabei ist es besonders bevorzugt, wenn mindestens zwei freie Hydroxy-Gruppen oder Carbonsäure-Gruppen unmittelbar an einen aromatischen Ring gebunden sind und ganz besonders bevorzugt in meta-Stellung zueinander angeordnet sind. Es ist weiterhin bevorzugt, dass die aromatischen Alkohole und Carbonsäuren ausgewählt sind aus den Phenolen, den Kresolen (o-, m- und p-Kresol), den Naphtholen (a- und β-Naphthol) und Thymol, sowie aus den Ethylphenolen, Propylphenolen, Fluorphenolen und Methoxyphenolen sowie Trimesinsäure und deren Ester, Gallussäure und deren Ester, Terephthalsäure und deren Ester, Phthalsäure und deren Ester und Phthalsäureanhydrid sowie Mischungen davon. Die Alkohole und Carbonsäuren können auch in Form ihrer Salze vorliegen, als Alkoholat bzw. Carboxylat.

Erfindungsgemäß bevorzugte aromatische Alkohole sind außerdem solche, die bei der Herstellung von Polycarbonat-Kunststoffen (z.B. für Compact Discs, Plastikschüsseln, Babyfläschchen) und Epoxydharzlacken (z.B. für Beschichtungen von Konservendosen und Folienverpackungen) verwendet werden, insbesondere 2,2-Bis-(4-hydroxyphenyl)-propan (Bisphenol A).

Ganz besonders bevorzugt ist es, wenn ein aromatischer Alkohol ausgewählt ist aus den Phenolen mit zwei oder mehr Hydroxygruppen, vorzugsweise aus 2,2-Bis-(4-hydroxyphenyl)-propan (Bisphenol A), Brenzcatechin, Resorcin, Hydrochinon und 1,4-Naphthohydrochinon, Phloroglucin, Pyrogallol, Hydroxyhydrochinon, wobei insbesondere Resorcin und/oder Phloroglucin als aromatische Alkohole bevorzugt sind.

In einer Ausführungsform gelangt man zu den erfindungsgemäßen Mikrokapseln, in dem der aromatische Alkohol als Ether eingesetzt wird, wobei der Ether in einer bevorzugten Ausführungsform ein Derivat der jeweiligen freien Form des erfindungsgemäß umzusetzenden aromatischen Alkohols ist. Der freie Alkohol kann dabei ebenso vorhanden sein; dann liegt demnach eine Mischung vor. Für diesen Fall kann das molare Verhältnis zwischen der freien Form des erfindungsgemäß umzusetzenden aromatischem Alkohols und der genannten zusätzlichen Komponente (Etherform eines aromatischen Alkohols) zwischen 0:100, bevorzugt, bevorzugt 1:1, oder 1:2 oder 1:4 betragen.

Der Vorteil der Mischung des aromatischen Alkohols mit einer Ether-Form ist darin begründet, dass damit die Reaktivität des Systems beeinflussbar ist. Insbesondere lässt sich mit der geeigneten Auswahl des Verhältnisses ein System schaffen, dessen Reaktivität in einem ausgewogenen Verhältnis zu der Lagerstabilität des Systems steht.

Als Ester der aromatischen Alkohole sind solche bevorzugt, welche unter den Polykondensationsbedingungen keine Nebenreaktionen eingehen und für den elektrophilen Angriff der eingesetzten aliphatischen und aromatischen Aldehyde genügend Reaktivität besitzen, um in hoher Ausbeute Polykondensationsprodukte zu erzeugen. Insbesondere die Ester von Carbonsäuren, Sulfonsäuren, Phosphorsäuren und Phosphonsäuren, aber auch die welche mit längeren Kohlenstoffketten einstellbare Grenzflächenaktivität besitzen sind von besonderem Interesse.

Als Estergruppen geeignet sind z.B. gesättigte oder ungesättigte, geradkettige, verzweigte oder cylische Kohlenwasserstoffreste sind, welche eines oder mehrere Heteroatome wie N, O, S, P, F, Cl, Br, I enthalten können, z.B. die Ester der Ameisensäure und ihrer Salze, Essigsäure und ihrer Salze, Propionsäure und ihrer Salze sowie Ester von C₆-C₁₄-Carbonsäuren und deren Salzen, Sulfonsäureester, z.B. para-Toluolsulfonsäureester, Amidosulfonsäureester, Phosphorsäureester, alle auf Basis der oben genannten aromatischen und heteroaromatischen Alkohole und Carbonsäuren.

In dieser Beschreibung bezeichnet der Begriff "aromatisch" (allein oder in Verbindung mit anderen Begriffen) ein mono- oder poylcyclischen (z.B. 2 oder 3 Ringe) aromatisches Ringsystem, vorzugsweise mit 6 bis 14 Ringatomen, z.B. Benzol oder Naphthalin. Der Begriff "heteroaromatisch" (allein oder in Verbindung mit anderen Begriffen) bezeichnet ein aromatisches heterocyclisches Ringsystem, vorzugsweise mit 5 bis 14 Ringatomen. Der Heteroaromat kann mono- oder polycyclisch (z.B. 2 oder 3 Ringe) sein. Beispiele für heteroaromatische Amine a1) sind Indol, Pyrol, Pyridin, Pyrimidin, Pyrazin, Triazin und Chinolin. Beispiele für heteroaromatische Verbindungen a2) sind Furan, Benzofuran, Thiophen, Benzothiophen, Pyran und Benzopyran.

Als Aldehyde b) mit mindestens 2 C-Atomen sind gemäß der vorliegenden Erfindung sowohl aliphatische als auch aromatische Aldehyde bevorzugt. Als aldehydische Komponente b) können auch Aldehydvorläufer (latente Aldehyde) wie Acetale und Halbacetale eingesetzt werden.

Besonders bevorzugte Aldehyde sind eine oder mehrere ausgewählt aus der folgenden Gruppe Valeraldehyd, Capronaldehyd, Caprylaldehyd, Decanal, Succindialdehyd, Cyclohexancarbaldehyd, Cyclopentancarbaldehyd, 2-Methyl-1-propanal, 2-Methylpropionaldehyd, Acetaldehyd, Acrolein, Aldosteron, Antimycin A, 8'-Apo-β-caroten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzaldehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd. Bevorzugt sind ebenfalls die Aldehydvorläufer (latente Aldehyde) der vorgenannten Aldehyde, z.B. deren Acetale und Halbacetale.

Im Sinne der vorliegenden Erfindung kann die aldehydische Komponente mindestens ein oder zwei, besonders bevorzugt zwei, drei oder vier, ganz besonders bevorzugt zwei freie Aldehyd-Gruppen pro Molekül, aufweisen, wobei es bevorzugt ist, wenn als aldehydische Komponente Glyoxal, Glyoxylsäure, Glutardialdehyd und/oder Succindialdehyd vorliegt, insbesondere Glyoxal, Glutardialdehyd und/oder Succindialdehyd, besonders bevorzugt ist Glutardialdehyd.

In den erfindungsgemäßen Mikrokapseln kann das molare Verhältnis von a) dem mindestens einen Amin und/oder aromatischen oder heteroaromatischen Verbindung (z.B. aromatischen Alkohol oder dessen Ether oder Derivat wie Ester), zu b) der mindestens einen aldehydischen Komponente im Allgemeinen zwischen 1:1 und 1:5, besonders bevorzugt zwischen 1:1 und 1:3 liegen. Bevorzugt liegt das Verhältnis bei Resorcin als Komponente a) bei etwa 1:1,5 bis 1:3, bei Phloroglucin bei etwa 1:1 bis 1:2, bei Melamin bei etwa 1:1,5 bis 1:2 und bei Harnstoff bei etwa 1:1,2 bis 1:1,5. Das Gewichtsverhältnis der Komponenten a) + b) zu c) (Schutzkolloid), d.h. das Verhältnis der Gewichtssumme von a) + b)) zum Gewicht der Komponente c) liegt im Allgemeinen zwischen 1:1 und 1:0,01 besonders bevorzugt zwischen 1 zu 0,2 und 1 zu 0,05.

Die im Rahmen der vorliegenden Erfindung verwendeten Copolymere c), enthalten Einheiten von 2-Acrylamido-2-methyl-propansulfonsäure oder dessen Salzen (AMPS, kommerziell z.B. erhältlich als Lupasol®PA 140, BASF) z.B. Alkalimetallsalzen wie Natrium- oder Kaliumsalzen oder Ammoniumsalzen, z.B. 2-Acrylamido-2-methyl-propansulfonsäurekaliumsalz oder 2-Acrylamido-2-methyl-propanphosphonsäure oder dessen Salzen z.B. Alkalimetallsalzen wie Natrium- oder Kaliumsalzen oder Ammoniumsalzen, und einem oder mehreren (Meth)Acrylaten. AMPS und AMPP können dabei auch in Mischung eingesetzt werden. Der Begriff "(Meth)Acrylat" bezeichnet in dieser Anmeldung sowohl Methacrylate wie Acrylate. Die Copolymere eigenen sich als Schutzkolloide und können vorteilhaft bei der Herstellung von Mikrokapseln eingesetzt werden.

Als Basismonomere eignen sich insbesondere:
2-Acrylamido-2-methyl-propansulfonsäure und dessen Alkali- und Ammonium-Salze 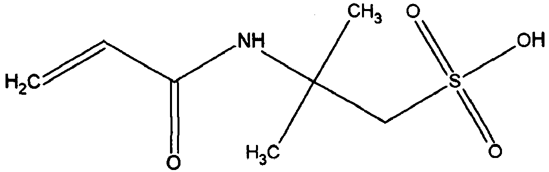
2-Acrylamido-2-methyl-1-propanphosphonsäure und dessen Alkali- und Ammonium-Salze:

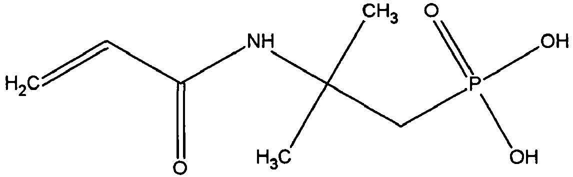

Besonders bevorzugt sind Copolymere c) auf Basis von AMPS. Die Copolymere c) können aus zwei oder mehr Comonomeren aufgebaut sein, beispielweise aus zwei Comonomeren (Bipolymere), drei Comonomeren (Terpolymere) oder aus vier Comonomeren. Es können neben AMPS und/oder AMPP ein, zwei oder mehr, insbesondere ein oder zwei (Meth)Acrylat-Comonomere enthalten sein.

Es sind neben AMPS und/oder AMPP, ein oder mehrere (Meth)Acrylat-Monomere enthalten und optional ein oder mehrere weitere Monomere, z.B. Acrylamid, N-Vinylpyrrolidon (kommerziell erhältlich als Luviskol®K15, K30 oder K90, BASF), Di- oder Polycarboxylate oder Polystyrolsulfonate, Vinylverbindungen wie Vinylester, Styrole, Vinylether, N-Vinylcaprolactam, Vinylphosphorsäure und deren Salze und Ester, Vinylphosphonsäure und deren Salze und Ester, Vinylsulfonsäure und deren Salze und Ester, Vinylcarbonsäuren und deren Salze und Ester (z.B. Vinylessigsäure) und/oder Maleinsäureanhydrid, Ethylen und/oder Maleinsäureanhydrid, Isobutylen und/oder Maleinsäureanhydrid, Styrol-Maleinsäureanhydrid, oder Salze von Amylverbindungen oder Allylverbindungen.

Als (Meth)Acrylat-Comonomere bevorzugt sind Acrylsäure und Methacrylsäure und deren Ester, wobei die Estergruppen z.B. gesättigte oder ungesättigte, geradkettige, verzweigte oder cylische Kohlenwasserstoffreste sind, welche eines oder mehrere Heteroatome wie N, O, S, P, F, Cl, Br, I enthalten können. Beispiele solcher Kohlenwasserstoffreste sind geradkettiges, verzweigtes oder cylisches Alkyl, geradkettiges, verzweigtes oder cylisches Alkenyl, Aryl wie Phenyl oder Heterocylyl wie Tetrahydrofurfuryl.

Als (Meth)Acrylat-Comonomere, kommen besonders bevorzugt in Frage:
a) Acrylsäure, C₁-C₁₄-Alkyl-Acrylsäure wie Methacrylsäure,
b) (Meth)Acrylamide wie Acrylamid, Methacrylamid, Diaceton-Acrylamid, Diaceton-Methacrylamid, N-Butoxymethyl-Acrylamid, N-iso-Butoxymethyl-Acrylamid, N-Butoxymethyl-Methacrylamid, N-iso-Butoxymethyl-Methacrylamid, N-MethylolAcrylamid, N-Methylol-Methacrylamid;
c) Heterocyclyl-(Meth)Acrylate wie Tetrahydrofurfuryl-acrylat und Tetrahydrofurfurylmethacrylat oder carbocyclische (Meth)Acrylate wie Isobornyl-acrylat und Isobornylmethacrylat,
d) Urethan(Meth)Acrylate wie Diurethandiacrylat und Diurethanmethacrylat (CAS: 72869-86-4)
e) C₁-C₁₄-Alkylacrylate wie Methyl-, Ethyl-, n-Propyl-, iso-Propyl-, n-Butyl-, sec. Butyl-, iso-Butyl-, tert. Butyl-, n-Pentyl-, iso-Pentyl-, Hexyl- (z.B. n-Hexyl, iso-Hexyl oder Cyclohexyl), Heptyl-, Octyl- (z.B. 2-Ethylhexyl), Nonyl-, Decyl- (z.B. 2-Propylheptyl oder iso-Decyl), Undecyl-, Dodecyl-, Tridecyl- (z.B. iso-Tridecyl), und Tetradecyl-Acrylat; die Alkylgruppen können optional mit einem oder mehreren Halogenatomen (z.B. Fluor, Chlor, Brom oder Iod) substituiert sein, z.B. Trifluoroethyl-Acrylat, oder mit einer oder mehreren Aminogruppen, z.B. Diethylaminoethyl-Acrylat, oder mit einer oder mehreren Alkoxygruppen wie Methoxypropyl-Acrylat, oder mit einer oder mehreren Aryloxygruppen wie Phenoxyethyl-Acrylat.
f) C₂-C₁₄-Alkenylacrylate wie Ethenyl-, n-Propenyl-, iso-Propenyl-, n-Butenyl-, sec. Butenyl-, iso-Butenyl-, tert. Butenyl-, n-Pentenyl-, iso-Pentenyl-, Hexenyl- (z.B. n-Hexenyl, iso-Hexenyl oder Cyclohexenyl), Heptenyl-, Octenyl- (z.B. 2-Ethylhexenyl), Nonenyl-, Decenyl- (z.B. 2-Propenylheptyl oder iso-Decenyl), Undecenyl-, Dodecenyl-, Tridecenyl- (z.B. iso-Tridecenyl), und Tetradecenyl-Acrylat, und deren Epoxide wie Glycidyl-Acrylat oder Aziridine wie Aziridin-Acrylat.
g) C₁-C₁₄- Hydroxyalkylacrylate wie Hydroxymethyl-, Hydroxyethyl-, Hydroxy-n-Propyl-, Hydroxy-iso-Propyl-, Hydroxy-n-Butyl-, Hydroxy-sec.-Butyl-, Hydroxy-iso-Butyl-, Hydroxy-tert.-Butyl-, Hydroxy-n-Pentyl-, Hydroxy-iso-Pentyl-, Hydroxyhexyl- (z.B. Hydroxy-n-Hexyl, Hydroxy-iso-Hexyl oder Hydroxy-Cyclohexyl), Hydroxyheptyl-, Hydroxyoctyl- (z.B. 2-Ethylhexyl), Hydroxynonyl-, Hydroxydecyl- (z.B. Hydroxy-2-Propylheptyl oder Hydroxy-iso-Decyl), Hydroxyundecyl-, Hydroxydodecyl-, Hydroxytridecyl- (z.B. Hydroxy-iso-Tridecyl), und Hydroxytetradecyl-Acrylat, wobei sich die Hydroxy-Gruppe bevorzugt in endständiger Position (ω-Position) (z.B. 4-Hydroxy-n-butylacrylat) oder in (ω-1)-Position (z.B. 2-Hydroxy-n-propylacrylat) des Alkylrests befindet;
h) Alkylenglykolacrylate, die eine oder mehrere Alkylenglykol-Einheiten enthalten. Beispiele sind i) Monoalkylenglykolacrylate, wie Acrylate von Ethylenglykol, Propylenglykol (z.B. 1,2- oder 1,3-Propandiol), Butylenglykol (z.B. 1,2-, 1,3- oder 1,4-Butandiol, Pentylenglykol (z.B. 1,5-Pentandiol) oder Hexylenglykol (z.B. 1,6-Hexandiol), bei denen die zweite Hydroxy-Gruppe verethert oder verestert ist, z.B. durch Schwefelsäure, Phosphorsäure, Acrylsäure oder Methacrylsäure, oder ii) Polyalkylenglykolacrylate wie Polyethylenglykolacrylate, Polypropylenglykolacrylate, Polybutylenglykolacrylate, Polypentylenglykolacrylate oder Polyhexylenglykolacrylate, deren zweite Hydroxy-Gruppe optional verethert oder verestert sein kann, z.B. durch Schwefelsäure, Phosphorsäure, Acrylsäure oder Methacrylsäure;
   Beispiele von (Poly)Alkylenglykol-Einheiten mit veretherten Hydroxygruppen sind C₁-C₁₄-Alkyloxy-(poly)alkylenglykole (z.B. C₁-C₁₄-Alkyloxy-(poly)alkylenglykolacrylate), Beispiele von (Poly)Alkylenglykol-Einheiten mit veresterten Hydroxygruppen sind Sulfonium-(poly)alkylenglykole (z.B. Sulfonium-(poly)alkylenglykolacrylate) und deren Salze, (Poly)Alkylenglykoldiacrylate wie 1,4-Butandioldiacrylat oder 1,6-Hexandioldiacrylat oder (Poly)Alkylenglykolmethacrylatacrylate wie 1,4-Butandiolmethacrylatacrylat oder 1,6-Hexandiolmethacrylatacrylat;
   Die Polyalkylenglykolacrylate können eine Acrylatgruppe tragen (z.B. Polyethylenglykolmonoacrylat, Polypropylenglykolmonoacrylat, Polybutylenglykolmonoacrylat, Polypentylenglykolmonoacrylat oder Polyhexylenglykolmonoacrylat) oder zwei oder mehr, bevorzugt zwei, Acrylatgruppen tragen wie Polyethylenglykoldiacrylat, Polypropylenglykoldiacrylat, Polybutylenglykoldiacrylat, Polypentylenglykoldiacrylat oder Polyhexylenglykoldiacrylat;
   Die Polyalkylenglykolacrylate können auch zwei oder mehr voneinander verschiedene Polyalkylenglykol-Blöcke enthalten, z.B. Blöcke von Polymethylenglykol und Polyethylenglykol oder Blöcke von Polyethylenglykol und Polypropylenglykol;
   Der Polymerisationsgrad der Polyalkylenglykol-Einheiten oder Polyalkylenglykol-Blöcke liegt im Allgemeinen im Bereich von 1 bis 20, vorzugsweise im Bereich von 3 bis 10, besonderes bevorzugt im Bereich von 3 bis 6.
i) C₁-C₁₄-Alkylmethacrylate wie Methyl-, Ethyl-, n-Propyl-, iso-Propyl-, n-Butyl-, sec. Butyl-, iso-Butyl-, tert. Butyl-, n-Pentyl-, iso-Pentyl-, Hexyl- (z.B. n-Hexyl, iso-Hexyl oder Cyclohexyl), Heptyl-, Octyl- (z.B. 2-Ethylhexyl), Nonyl-, Decyl- (z.B. 2-Propylheptyl oder iso-Decyl), Undecyl-, Dodecyl-, Tridecyl- (z.B. iso-Tridecyl), und Tetradecyl-Methacrylat; die Alkylgruppen können optional mit einem oder mehreren Halogenatomen (z.B. Fluor, Chlor, Brom oder Iod) substituiert sein, z.B. Trifluoroethyl-Methacrylat, oder mit einer oder mehreren Aminogruppen, z.B. DiethylaminoethylMethacrylat, oder mit einer oder mehreren Alkoxygruppen wie Methoxypropyl-Methacrylat, oder mit einer oder mehreren Aryloxygruppen wie PhenoxyethylMethacrylat.
j) C₂-C₁₄-Alkenylmethacrylate wie Ethenyl-, n-Propenyl-, iso-Propenyl-, n-Butenyl-, sec. Butenyl-, iso-Butenyl-, tert. Butenyl-, n-Pentenyl-, iso-Pentenyl-, Hexenyl- (z.B. n-Hexenyl, iso-Hexenyl oder Cyclohexenyl), Heptenyl-, Octenyl- (z.B. 2-Ethylhexenyl), Nonenyl-, Decenyl- (z.B. 2-Propenylheptyl oder iso-Decenyl), Undecenyl-, Dodecenyl-, Tridecenyl- (z.B. iso-Tridecenyl), und Tetradecenyl-Methacrylat, und deren Epoxide wie Glycidyl-Methacrylat oder Aziridine wie Aziridin-Methacrylat.
k) C₁-C₁₄-Hydroxyalkylmethacrylate wie Hydroxymethyl-, Hydroxyethyl-, Hydroxy-n-Propyl-, Hydroxy-iso-Propyl-, Hydroxy-n-Butyl-, Hydroxy-sec.-Butyl-, Hydroxy-iso-Butyl-, Hydroxy-tert.-Butyl-, Hydroxy-n-Pentyl-, Hydroxy-iso-Pentyl-, Hydroxyhexyl- (z.B. Hydroxy-n-Hexyl, Hydroxy-iso-Hexyl oder Hydroxy-Cyclohexyl), Hydroxyheptyl-, Hydroxyoctyl- (z.B. 2-Ethylhexyl), Hydroxynonyl-, Hydroxydecyl- (z.B. Hydroxy-2-Propylheptyl oder Hydroxy-iso-Decyl), Hydroxyundecyl-, Hydroxydodecyl-, Hydroxytridecyl- (z.B. Hydroxy-iso-Tridecyl), und Hydroxytetradecyl-Methacrylat, wobei sich die Hydroxy-Gruppe bevorzugt in endständiger Position (ω-Position) (z.B. 4-Hydroxy-n-butylmethacrylat) oder in (ω-1)-Position (z.B. 2-Hydroxy-n-propylmethacrylat) des Alkylrests befindet;
l) Alkylenglykolmethacrylate, die eine oder mehrere Alkylenglykol-Einheiten enthalten. Beispiele sind i) Monoalkylenglykolmethacrylate, wie Methacrylate von Ethylenglykol, Propylenglykol (z.B. 1,2- oder 1,3-Propandiol), Butylenglykol (z.B. 1,2-, 1,3- oder 1,4-Butandiol, Pentylenglykol (z.B. 1,5-Pentandiol) oder Hexylenglykol (z.B. 1,6-Hexandiol), bei denen die zweite Hydroxy-Gruppe verethert oder verestert ist, z.B. durch Schwefelsäure, Phosphorsäure, Acrylsäure oder Methacrylsäure, oder ii) Polyalkylenglykolmethacrylate wie Polyethylenglykolmethacrylate, Polypropylenglykolmethacrylate, Polybutylenglykolmethacrylate, Polypentylenglykolmethacrylate oder Polyhexylenglykolmethacrylate, deren zweite Hydroxy-Gruppe optional verethert oder verestert sein kann, z.B. durch Schwefelsäure, Phosphorsäure, Acrylsäure oder Methacrylsäure;
   Beispiele von (Poly)Alkylenglykol-Einheiten mit veretherten Hydroxygruppen sind C₁-C₁₄-Alkyloxy-(poly)alkylenglykole (z.B. C₁-C₁₄-Alkyloxy-(poly)alkylenglykolmethacrylate), Beispiele von (Poly)Alkylenglykol-Einheiten mit veresterten Hydroxygruppen sind Sulfonium-(poly)alkylenglykole (z.B. Sulfonium-(poly)alkylenglykolmethacrylate) und deren Salze oder (Poly)Alkylenglykoldimethacrylate wie 1,4-Butandioldimethacrylat;
   Die Polyalkylenglykolmethacrylate können eine Methacrylatgruppe tragen (z.B. Polyethylenglykolmonomethacrylat, Polypropylen-glykolmonomethacrylat, Polybutylenglykol-monomethacrylat, Polypentylenglykolmono-methacrylat oder Polyhexylenglykolmonomethacrylat) zwei oder mehr, bevorzugt zwei, Methacrylatgruppen tragen, wie Polyethylenglykoldimethacrylat, Polypropylenglykoldimethacrylat, Polybutylenglykoldimethacrylat, Polypentylenglykoldi-methacrylat oder Polyhexylenglykoldimethacrylat;
   Die Polyalkylenglykolmethacrylate können auch zwei oder mehr voneinander verschiedene Polyalkylenglykol-Blöcke enthalten, z.B. Blöcke von Polymethylenglykol und Polyethylenglykol oder Blöcke von von Polyethylenglykol und Polypropylenglykol (z.B. Bisomer PEM63PHD (Cognis), CAS 58916-75-9);
   Der Polymerisationsgrad der Polyalkylenglykol-Einheiten oder Polyalkylenglykol-Blöcke liegt im Allgemeinen im Bereich von 1 bis 20, vorzugsweise im Bereich von 3 bis 10, besonderes bevorzugt im Bereich von 3 bis 6.

Beispiele bevorzugter (Meth)Acrylat-Comonomere sind nachfolgend aufgeführt:
a) 4-Hydroxybutylacrylat 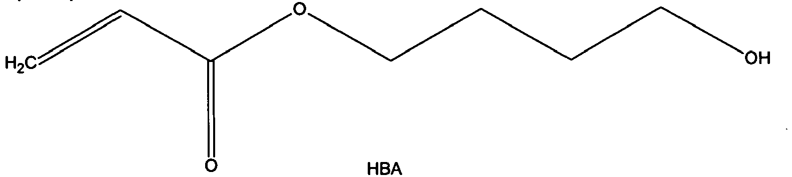
b) 2-Hydroxypropylmethacrylat 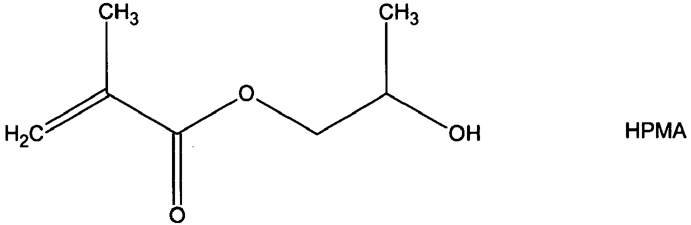
c) Ammoniumsulfatoethylmethacrylat 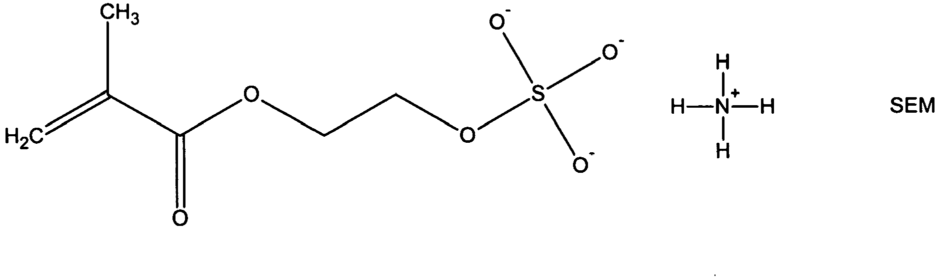
d) Pentapropylenglykolmethacrylat 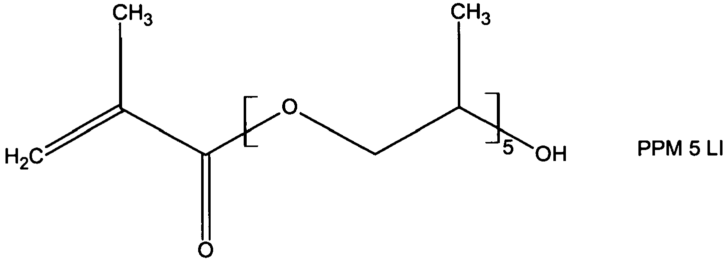
e) Acrylsäure 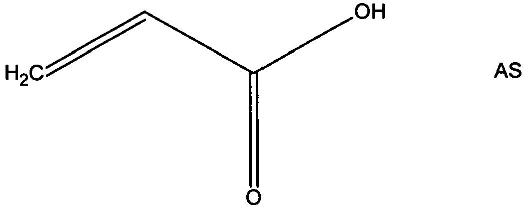
f) Hexaethylenglykolmethyacrylat 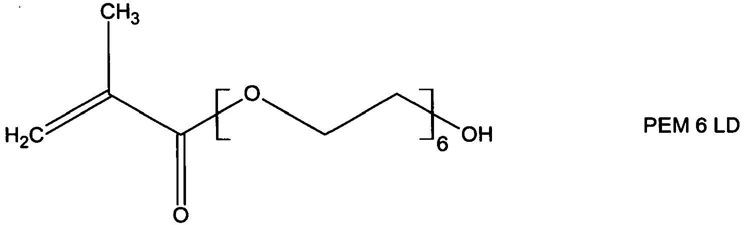
g) Hexapropylenglykolacrylat 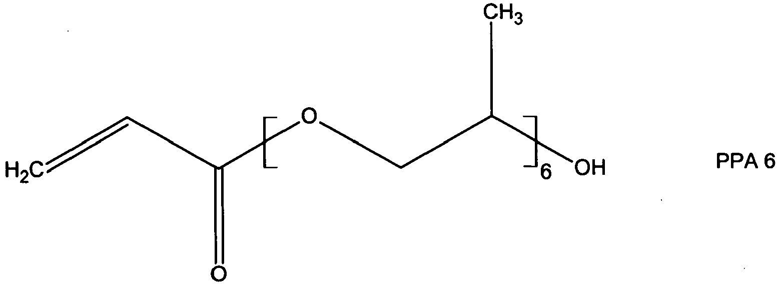
h) Hexaethylenglykolacrylat 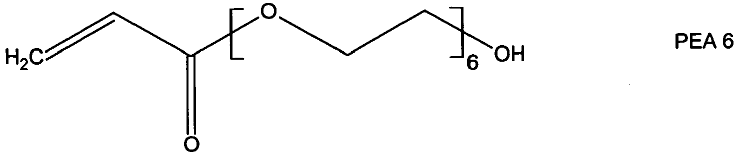
i) Hydroxyethylmethacrylat 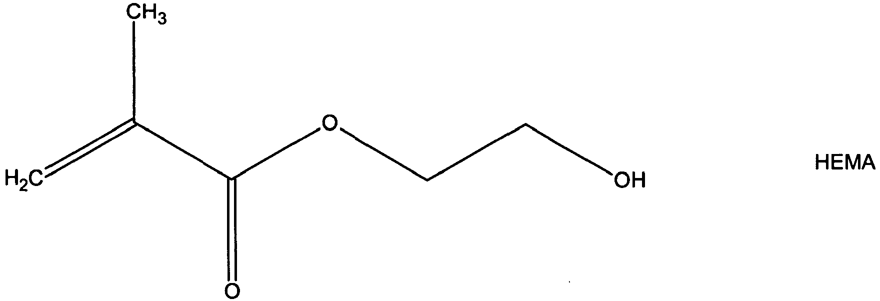
j) Polyalkylenglycolmethacrylat (CAS-Nr. 589-75-9) 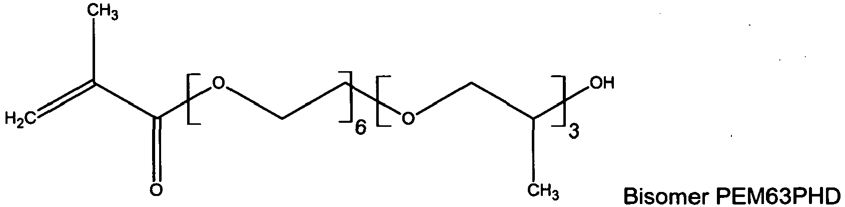
k) Methoxy-polyethylenglycolmethacrylat 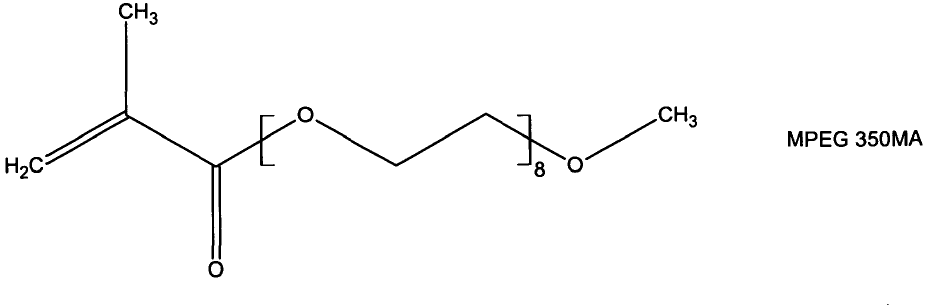
I) 2-Propylheptylacrylat (2-PHA) 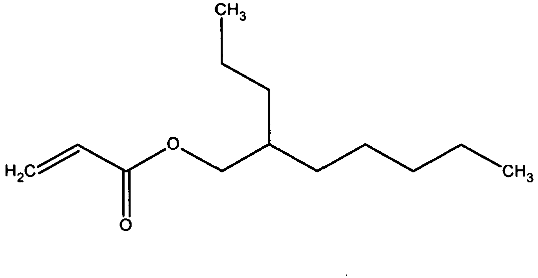
m) 1,3-Butandioldimethacrylat (BDDMA) 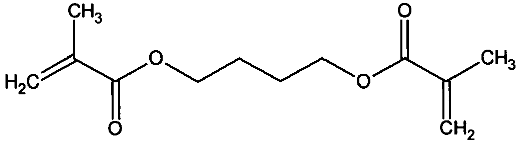
n) Triethylenglykoldimethacrylat (TEGDMA) 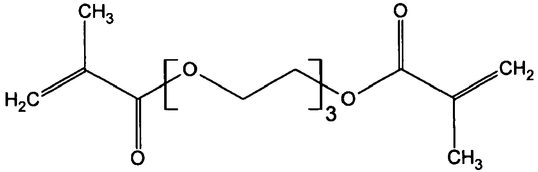
o) Hydroxyethylacrylat (HEA) 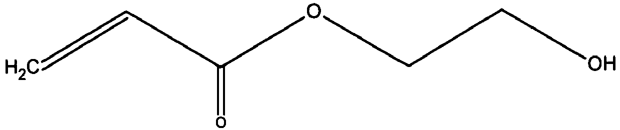
p) 2-Hydroxypropylacrylat (HPA) 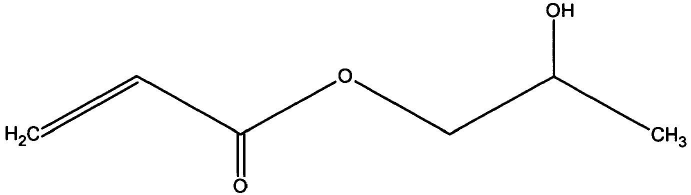
q) Ethylenglykoldimethacrylat (EGDMA) 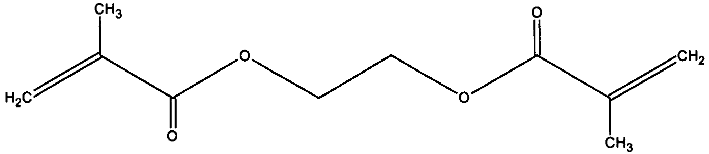
r) Glycidylmethacrylat (GMA) 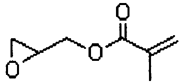
s) Allylmethacrylat (ALMA) 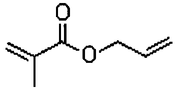
t) Bisomer PEM 3 (Polyethylenglykolmethacrylat) 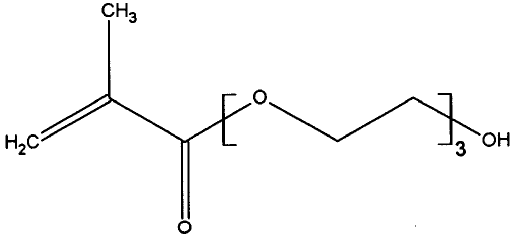
u) Bisomer IDMA (Isodecylmethacrylat) 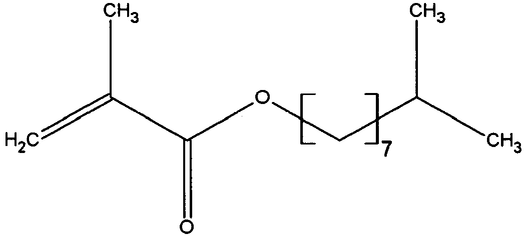
v) Bisomer C13MA (Isotridecylmethacrylat) 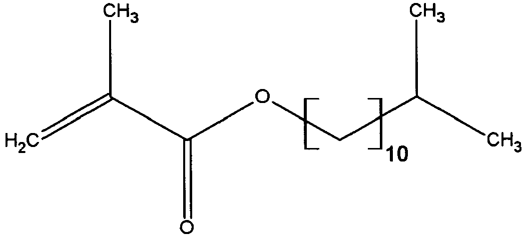
w) (3-Sulfopropyl)-acrylat, insbesondere als Salz wie das Kaliumsalz 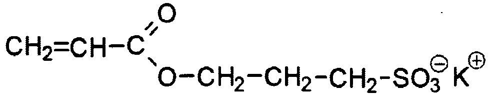
x) N-(2-Acryloyloxyethyl)-N,N-dimethyl-N-(3-sulfopropyl)ammoniumbetain 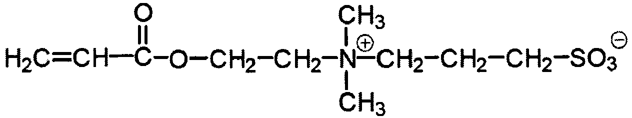
y) N-(2-Methacryloyloxyethyl)-N,N-dimethyl-N-(3-sulfopropyl)ammoniumbetain 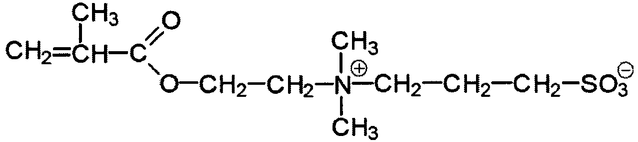
z) (3-Sulfopropyl)-methacrylat, insbesondere als Salz wie das Kaliumsalz 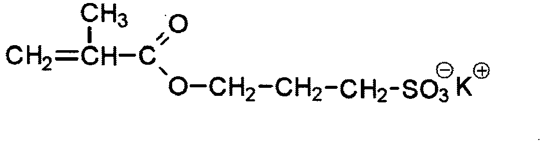
z1) N-(3-Methacrylamidopropyl)-N,N-dimethyl-N-(3-sulfopropyl)ammoniumbetain

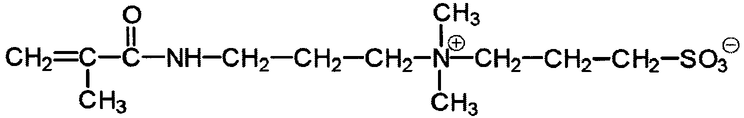

Bevorzugte Copolymere c) sind Bipolymere enthaltend AMPS und eines der vorgenannten Comonomere a) bis z1). Bevorzugt sind auch Bipolymere enthaltend AMPP und eines der vorgenannten Comonomere a) bis z1).

Auch Terpolymere von AMPS oder AMPP mit zwei (Meth)Acrylat-Monomeren sind einsetzbar.

Sind in den Copolymeren c) neben AMPS und/oder AMPP und (Meth)acrylaten weitere Monomere enthalten, sind diese bevorzugt ausgewählt aus der Gruppe der Vinylverbindungen wie Vinylester, Styrole, N-Vinylcaprolactam, Vinylphosphorsäure und deren Salze und Ester, Vinylphosphonsäure und deren Salze und Ester, Vinylsulfonsäure und deren Salze und Ester, Vinylcarbonsäuren und deren Salze und Ester (z.B. Vinylessigsäure), ungesättigen Di- oder Polycarbonsäuren wie Maleinsäureester, oder Salze von Amylverbindungen oder Allylverbindungen. Nachfolgend werden bevorzugte weitere Comonomere für AMPS, AMPP und (Meth)Acrylatmonomere genannt:
1) Vinylverbindungen, z.B. Vinylester wie Vinylacetat, Vinyllaurat, Vinylpropionat oder Vinylester der Neononansäure, N-Vinylcaprolactam, Vinylphosphorsäure und deren Salze und Ester, Vinylphosphonsäure und deren Salze und Ester, Vinylsulfonsäure und deren Salze und Ester, Vinylcarbonsäuren und deren Salze und Ester (z.B. Vinylessigsäure), oder aromatische Vinylverbindungen wie Styrol-Comonomere, beispielsweise Styrol, alpha-Methylstyrol oder polar funktionalisierte Styrole wie Styrole mit Hydroxy-, Amino-, Nitril-, Carbonsäure-, Phosphonsäure-, Phosphorsäure-, Nitro- oder Sulfonsäure-Gruppen und deren Salze, wobei die Styrole bevorzugt in para-Position polar funktionalisiert sind,.
2) Ungesättigte Di- oder Polycarbonsäuren, z.B. Maleinsäureester wie Dibutylmaleinat oder Dioctylmaleinat, als Salze von Allylverbindungen z.B. Natriumallylsulfonat, als Salze von Amylderivaten z.B. Natriumamylsulfonat.

Bevorzugte Copolymere c) sind daher auch Terpolymere enthaltend AMPS, eines der vorgenannten Comonomere a) bis z1) und ein weiteres Comonomer ausgewählt aus der Gruppe der Vinylverbindungen wie Vinylester, Styrole, N-Vinylcaprolactam, Vinylphosphorsäure und deren Salze und Ester, Vinylphosphonsäure und deren Salze und Ester, Vinylsulfonsäure und deren Salze und Ester, Vinylcarbonsäuren und deren Salze und Ester (z.B. Vinylessigsäure), ungesättigen Di- oder Polycarbonsäuren wie Maleinsäureester, oder Salze von Amylverbindungen oder Allylverbindungen.

Bevorzugt sind auch Terpolymere enthaltend AMPP, eines der vorgenannten Comonomere a) bis z1) und ein weiteres Comonomer ausgewählt aus der Gruppe der Vinylverbindungen wie Vinylester, Styrole, N-Vinylcaprolactam, Vinylphosphorsäure und deren Salze und Ester, Vinylphosphonsäure und deren Salze und Ester, Vinylsulfonsäure und deren Salze und Ester, Vinylcarbonsäuren und deren Salze und Ester (z.B. Vinylessigsäure), ungesättigen Di- oder Polycarbonsäuren wie Maleinsäureester, oder Salze von Amylverbindungen oder Allylverbindungen.

Beispiele für besonders bevorzugte Terpolymere sind Terpolymere des Typs AMPS + HEMA (Comonomer i) + Vinylacetat oder Terpolymere des Typs AMPP + HEMA + Vinylacetat, AMPS + HEMA + Vinylpropionat, AMPP + HEMA + Vinylpropionat, AMPS + HEMA + N-Vinylcaprolactam, AMPP + HEMA + N-Vinylcaprolactam, AMPS + HEMA + Styrol, AMPP + HEMA + Styrol, AMPS + HEMA + p-Hydroxystyrol, AMPP + HEMA + p-Hydroxystyrol, AMPS + HEMA + Vinyllaurat, AMPP + HEMA + Vinyllaurat, AMPS + HEMA + Vinylphosphorsäure, AMPP + HEMA + Vinylphosphorsäure, AMPS + HEMA + Vinylphosphorsäuretrimethylester, AMPP + HEMA + Vinylphosphorsäuretrimethylester, AMPS + HEMA + Vinylphosphonsäure, AMPP + HEMA + Vinylphosphonsäure, AMPS + HEMA + Vinylphosphonsäuredimethylester, AMPP + HEMA + Vinylphosphonsäuredimethylester, AMPS + HEMA + Vinylsulfonsäure, AMPP + HEMA + Vinylsulfonsäure, AMPS + HEMA + Vinylessigsäure, AMPP + HEMA + Vinylessigsäure;
AMPS + HEA (Comonomer o) + Vinylacetat, AMPP + HEA + Vinylacetat, AMPS + HEA + Vinylpropionat, AMPP + HEA + Vinylpropionat, AMPS + HEA + N-Vinylcaprolactam, AMPP + HEA + N-Vinylcaprolactam, AMPS + HEA + Styrol, AMPP + HEA + Styrol, AMPS + HEA + p-Hydroxystyrol, AMPP + HEA + p-Hydroxystyrol, AMPS + HEA + Vinyllaurat, AMPP + HEA + Vinyllaurat, AMPS + HEA + Vinylphosphorsäure, AMPP + HEA + Vinylphosphorsäure, AMPS + HEA + Vinylphosphorsäuretrimethylester, AMPP + HEA + Vinylphosphorsäuretrimethylester, AMPS + HEA + Vinylphosphonsäure, AMPP + HEA + Vinylphosphonsäure, AMPS + HEA + Vinylphosphonsäuredimethylester, AMPP + HEA + Vinylphosphonsäuredimethylester, AMPS + HEA + Vinylsulfonsäure, AMPP + HEA + Vinylsulfonsäure, AMPS + HEA + Vinylessigsäure, AMPP + HEA + Vinylessigsäure;
AMPS + HPMA (Comonomer b) + Vinylacetat, AMPP + HPMA + Vinylacetat, AMPS + HPMA + Vinylpropionat, AMPP + HPMA + Vinylpropionat, AMPS + HPMA + N-Vinylcaprolactam, AMPP + HPMA + N-Vinylcaprolactam, AMPS + HPMA + Styrol, AMPP + HPMA + Styrol, AMPS + HPMA + p-Hydroxystyrol, AMPP + HPMA + p-Hydroxystyrol, AMPS + HPMA + Vinyllaurat, AMPP + HPMA + Vinyllaurat, AMPS + HPMA + Vinylphosphorsäure, AMPP + HPMA + Vinylphosphorsäure, AMPS + HPMA + Vinylphosphorsäuretrimethylester, AMPP + HPMA + Vinylphosphorsäuretrimethylester, AMPS + HPMA + Vinylphosphonsäure, AMPP + HPMA + Vinylphosphonsäure, AMPS + HPMA + Vinylphosphonsäuredimethylester, AMPP + HPMA + Vinylphosphonsäuredimethylester, AMPS + HPMA + Vinylsulfonsäure, AMPP + HPMA + Vinylsulfonsäure, AMPS + HPMA + Vinylessigsäure, AMPP + HPMA + Vinylessigsäure;
AMPS + HPA (Comonomer p) + Vinylacetat, AMPP + HPA + Vinylacetat, AMPS + HPA + Vinylpropionat, AMPP + HPA + Vinylpropionat, AMPS + HPA + N-Vinylcaprolactam, AMPP + HPA + N-Vinylcaprolactam, AMPS + HPA + Styrol, AMPP + HPA + Styrol, AMPS + HPA + p-Hydroxystyrol, AMPP + HPA + p-Hydroxystyrol, AMPS + HPA + Vinyllaurat, AMPP + HPA + Vinyllaurat, AMPS + HPA + Vinylphosphorsäure, AMPP + HPA + Vinylphosphorsäure, AMPS + HPA + Vinylphosphorsäuretrimethylester, AMPP + HPA + Vinylphosphorsäuretrimethylester, AMPS + HPA + Vinylphosphonsäure, AMPP + HPA + Vinylphosphonsäure, AMPS + HPA + Vinylphosphonsäuredimethylester, AMPP + HPA + Vinylphosphonsäuredimethylester, AMPS + HPA + Vinylsulfonsäure, AMPP + HPA + Vinylsulfonsäure, AMPS + HPA + Vinylessigsäure, AMPP + HPA + Vinylessigsäure;
AMPS + HBA (Comonomer a) + Vinylacetat, AMPP + HBA + Vinylacetat, AMPS + HBA + Vinylpropionat, AMPP + HBA + Vinylpropionat, AMPS + HBA + N-Vinylcaprolactam, AMPP + HBA + N-Vinylcaprolactam, AMPS + HBA + Styrol, AMPP + HBA + Styrol, AMPS + HBA + p-Hydroxystyrol, AMPP + HBA + p-Hydroxystyrol, AMPS + HBA + Vinyllaurat, AMPP + HBA + Vinyllaurat, AMPS + HBA + Vinylphosphorsäure, AMPP + HBA + Vinylphosphorsäure, AMPS + HBA + Vinylphosphorsäuretrimethylester, AMPP + HBA + Vinylphosphorsäuretrimethylester, AMPS + HBA + Vinylphosphonsäure, AMPP + HBA + Vinylphosphonsäure, AMPS + HBA + Vinylphosphonsäuredimethylester, AMPP + HBA + Vinylphosphonsäuredimethylester, AMPS + HBA + Vinylsulfonsäure, AMPP + HBA + Vinylsulfonsäure, AMPS + HBA + Vinylessigsäure, AMPP + HBA + Vinylessigsäure;
AMPS + PEM6LD (Comonomer f) + Vinylacetat, AMPP + PEM6LD + Vinylacetat, AMPS + PEM6LD + Vinylpropionat, AMPP + PEM6LD + Vinylpropionat, AMPS + PEM6LD + N-Vinylcaprolactam, AMPP + PEM6LD + N-Vinylcaprolactam, AMPS + PEM6LD + Styrol, AMPP + PEM6LD + Styrol, AMPS + PEM6LD + p-Hydroxystyrol, AMPP + PEM6LD + p-Hydroxystyrol, AMPS + PEM6LD + Vinyllaurat, AMPP + PEM6LD + Vinyllaurat, AMPS + PEM6LD + Vinylphosphorsäure, AMPP + PEM6LD + Vinylphosphorsäure, AMPS + PEM6LD + Vinylphosphorsäuretrimethylester, AMPP + PEM6LD + Vinylphosphorsäuretrimethylester, AMPS + PEM6LD + Vinylphosphonsäure, AMPP + PEM6LD + Vinylphosphonsäure, AMPS + PEM6LD + Vinylphosphonsäuredimethylester, AMPP + PEM6LD + Vinylphosphonsäuredimethylester, AMPS + PEM6LD + Vinylsulfonsäure, AMPP + PEM6LD + Vinylsulfonsäure, AMPS + PEM6LD + Vinylessigsäure, AMPP + PEM6LD + Vinylessigsäure;
AMPS + PPA6 (Comonomer g) + Vinylacetat, AMPP + PPA6 + Vinylacetat, AMPS + PPA6 + Vinylpropionat, AMPP + PPA6 + Vinylpropionat, AMPS + PPA6 + N-Vinylcaprolactam, AMPP + PPA6 + N-Vinylcaprolactam, AMPS + PPA6 + Styrol, AMPP + PPA6 + Styrol, AMPS + PPA6 + p-Hydroxystyrol, AMPP + PPA6 + p-Hydroxystyrol, AMPS + PPA6 + Vinyllaurat, AMPP + PPA6 + Vinyllaurat, AMPS + PPA6 + Vinylphosphorsäure, AMPP + PPA6 + Vinylphosphorsäure, AMPS + PPA6 + Vinylphosphorsäuretrimethylester, AMPP + PPA6 + Vinylphosphorsäuretrimethylester, AMPS + PPA6 + Vinylphosphonsäure, AMPP + PPA6 + Vinylphosphonsäure, AMPS + PPA6 + Vinylphosphonsäuredimethylester, AMPP + PPA6 + Vinylphosphonsäuredimethylester, AMPS + PPA6 + Vinylsulfonsäure, AMPP + PPA6 + Vinylsulfonsäure, AMPS + PPA6 + Vinylessigsäure, AMPP + PPA6 + Vinylessigsäure;
Möglich sind auch Tetrapolymere z.B. AMPS oder AMPP + HEMA + Vinylacetat, und höhere Copolymere durch Einbau weiterer Comonomere, je nach Eigenschafts- und Anforderungsprofil.

Die Copolymere c) weisen im Allgemeinen einen Anteil an AMPS-Einheiten oder AMPP-Einheiten von größer 50 Mol-% auf, vorzugsweise im Bereich von 60 - 95 Mol-%, besonders bevorzugt von 80 bis 99 Mol-%, der Anteil der weiteren Monomere liegt im Allgemeinen kleiner 50 Mol-%, vorzugsweise im Bereich von 5 bis 40 Mol-%, besonders bevorzugt von 0,1 bis 20 Mol-%. Der Anteil an (Meth)Acrylat-Monomeren liegt in Bipolymeren im Allgemeinen im Bereich von 0,1 Mol-% bis 40 Mol-%, in Terpolymeren im Bereich von 0,1 Mol-% bis 20 Mol-%. Der Anteil weiterer Monomere in Terpolymeren liegt im Allgemeinen im Bereich von 0,1 Mol-% bis 5 Mol-%.

Die Copolymere c) können durch an sich bekannte Verfahren erhalten werden, beispielsweise im Batch- oder im Semibatch-Verfahren. Beispielsweise werden zunächst entsprechende Mengen Wasser und Monomeren in einen temperierbaren Reaktor geleitet und unter Inertgas-Atmosphäre gesetzt. Die Vorlage dann gerührt, auf Reaktionstemperatur gebracht (vorzugsweise im Bereich von ca. 70 - 80 °C) und Initiator zugesetzt, vorzugsweise in Form einer wässrigen Lösung. Als Initiator eignen sich bekannte Initiatoren für radikalische Polymerisationen, beispielsweise Natrium-, Kalium- oder Ammoniumperoxodisulfat, oder H₂O₂ basierte Mischungen, z.B. Mischungen von H₂O₂ mit Zitronensäure. Die maximale Temperatur wird abgewartet und sobald die Temperatur im Reaktor sinkt erfolgt entweder a) die Zudosierung der restlichen Monomere und anschließend eine Nachreaktion (Semibatch-Verfahren), oder b) direkt die Nachreaktion (Batch-Verfahren). Danach wird das erhaltene Reaktionsgemisch auf Raumtemperatur abgekühlt und das Copolymer aus der wässrigen Lösung isoliert, z.B. durch Extraktion mit organischen Lösungsmitteln wie Hexan oder Methylenchlorid und anschließendes Abdestillieren des Lösungsmittels. Danach kann das Copolymer mit organischem Lösungsmittel gewaschen und getrocknet werden. Das erhaltene Reaktionsgemisch kann auch direkt weiterverarbeitet werden, in diesem Falle ist es von Vorteil, der wässrigen Copolymer-Lösung ein Konservierungsmittel zuzusetzen.

Die Copolymere eignen sich als Schutzkolloide bei der Herstellung von Mikrokapseln. Bevorzugte Mikrokapseln der vorliegenden Erfindung weisen folgende Komponenten a), b), und c) auf:
Phloroglucin, Glutardialdehyd, AMPS/Hydroxyethylmethacrylat-Copolymer;
   Phloroglucin, Succindialaldehyd, AMPS/Hydroxyethylmethacrylat-Copolymer;
   Phloroglucin, Glyoxal, AMPS/Hydroxyethylmethacrylat-Copolymer;
   Phloroglucin, Glyoxylsäure, AMPS/Hydroxyethylmethacrylat-Copolymer;
Phloroglucin, Glutardialdehyd, AMPS/Hydroxyethylacrylat-Copolymer;
   Phloroglucin, Succindialaldehyd, AMPS/Hydroxyethylacrylat-Copolymer;
   Phloroglucin, Glyoxal, AMPS/Hydroxyethylacrylat-Copolymer;
   Phloroglucin, Glyoxylsäure, AMPS/Hydroxyethylacrylat-Copolymer;
Phloroglucin, Glutardialdehyd, AMPS/Hydroxypropylmethacrylat-Copolymer;
   Phloroglucin, Succindialaldehyd, AMPS/Hydroxypropylmethacrylat-Copolymer;
   Phloroglucin, Glyoxal, AMPS/Hydroxypropylmethacrylat-Copolymer;
   Phloroglucin, Glyoxylsäure, AMPS/Hydroxypropylmethacrylat-Copolymer;
Phloroglucin, Glutardialdehyd, AMPS/Hydroxypropylacrylat-Copolymer;
   Phloroglucin, Succindialaldehyd, AMPS/Hydroxypropylacrylat-Copolymer;
   Phloroglucin, Glyoxal, AMPS/Hydroxypropylacrylat-Copolymer;
   Phloroglucin, Glyoxylsäure, AMPS/Hydroxypropylacrylat-Copolymer;
Phloroglucin, Glutardialdehyd, AMPS/Hydroxybutylmethacrylat-Copolymer;
   Phloroglucin, Succindialaldehyd, AMPS/Hydroxybutylmethacrylat-Copolymer;
   Phloroglucin, Glyoxal, AMPS/Hydroxybutylmethacrylat-Copolymer;
   Phloroglucin, Glyoxylsäure, AMPS/Hydroxbutylmethacrylat-Copolymer;
Phloroglucin, Glutardialdehyd, AMPS/Hydroxybutylacrylat-Copolymer;
   Phloroglucin, Succindialaldehyd, AMPS/Hydroxybutylacrylat-Copolymer;
   Phloroglucin, Glyoxal, AMPS/Hydroxybutylacrylat-Copolymer;
   Phloroglucin, Glyoxylsäure, AMPS/Hydroxybutylacrylat-Copolymer;
Phloroglucin, Glutardialdehyd, AMPS/Polyethylenglykolmonomethacrylat-Copolymer;
   Phloroglucin, Succindialaldehyd, AMPS/Polyethylenglykolmonomethacrylat-Copolymer;
   Phloroglucin, Glyoxal, AMPS/ Polyethylenglykolmonomethacrylat-Copolymer;
   Phloroglucin, Glyoxylsäure, AMPS/Polyethylenglykolmonomethacrylat-Copolymer;
Phloroglucin, Glutardialdehyd, AMPS/Polyethylenglykolmonoacrylat-Copolymer;
   Phloroglucin, Succindialaldehyd, AMPS/Polyethylenglykolmonoacrylat-Copolymer;
   Phloroglucin, Glyoxal, AMPS/ Polyethylenglykolmonoacrylat-Copolymer;
   Phloroglucin, Glyoxylsäure, AMPS/ Polyethylenglykolmonoacrylat-Copolymer;
Phloroglucin, Glutardialdehyd, AMPS/Polypropylenglykolmonomethacrylat-Copolymer;
   Phloroglucin, Succindialaldehyd, AMPS/Polypropylenglykolmonomethacrylat-Copolymer;
   Phloroglucin, Glyoxal, AMPS/ Polypropylenglykolmonomethacrylat-Copolymer;
   Phloroglucin, Glyoxylsäure, AMPS/ Polypropylenglykolmonomethacrylat-Copolymer;
Phloroglucin, Glutardialdehyd, AMPS/Polypropylenglykolmonoacrylat-Copolymer;
   Phloroglucin, Succindialaldehyd, AMPS/Polypropylenglykolmonoacrylat-Copolymer;
   Phloroglucin, Glyoxal, AMPS/ Polypropylenglykolmonoacrylat-Copolymer;
   Phloroglucin, Glyoxylsäure, AMPS/ Polypropylenglykolmonoacrylat-Copolymer;
Phloroglucin, Glutardialdehyd, AMPS/Methoxy-polyethylenglykolmonomethacrylat-Copolymer;
   Phloroglucin, Succindialaldehyd, AMPS/Methoxy-polyethylenglykolmonomethacrylat-Copolymer;
   Phloroglucin, Glyoxal, AMPS/ Methoxy-polyethylenglykolmonomethacrylat-Copolymer;
   Phloroglucin, Glyoxylsäure, AMPS/ Methoxy-polyethylenglykolmonomethacrylatCopolymer;
Phloroglucin, Glutardialdehyd, AMPS/Methoxy-polyethylenglykolmonoacrylat-Copolymer;
   Phloroglucin, Succindialaldehyd, AMPS/Methoxy-polyethylenglykolmonoacrylat-Copolymer;
   Phloroglucin, Glyoxal, AMPS/ Methoxy-polyethylenglykolmonoacrylat-Copolymer;
   Phloroglucin, Glyoxylsäure, AMPS/ Methoxy-polyethylenglykolmonoacrylat-Copolymer;
Resorcin, Glutardialdehyd, AMPS/Hydroxyethylmethacrylat-Copolymer;
   Resorcin, Succindialaldehyd, AMPS/Hydroxyethylmethacrylat-Copolymer;
   Resorcin, Glyoxal, AMPS/Hydroxyethylmethacrylat-Copolymer;
   Resorcin, Glyoxylsäure, AMPS/Hydroxyethylmethacrylat-Copolymer
Resorcin, Glutardialdehyd, AMPS/Hydroxyethylacrylat-Copolymer;
   Resorcin, Succindialaldehyd, AMPS/Hydroxyethylacrylat-Copolymer;
   Resorcin, Glyoxal, AMPS/Hydroxyethylacrylat-Copolymer;
   Resorcin, Glyoxylsäure, AMPS/Hydroxyethylacrylat-Copolymer
Resorcin, Glutardialdehyd, AMPS/Hydroxypropylmethacrylat-Copolymer;
   Resorcin, Succindialaldehyd, AMPS/Hydroxypropylmethacrylat-Copolymer;
   Resorcin, Glyoxal, AMPS/Hydroxypropylmethacrylat-Copolymer;
   Resorcin, Glyoxylsäure, AMPS/Hydroxypropylmethacrylat-Copolymer
Resorcin, Glutardialdehyd, AMPS/Hydroxypropylacrylat-Copolymer;
   Resorcin, Succindialaldehyd, AMPS/Hydroxypropylacrylat-Copolymer;
   Resorcin, Glyoxal, AMPS/Hydroxypropylacrylat-Copolymer;
   Resorcin, Glyoxylsäure, AMPS/Hydroxypropylacrylat-Copolymer
Resorcin, Glutardialdehyd, AMPS/Hydroxybutylmethacrylat-Copolymer;
   Resorcin, Succindialaldehyd, AMPS/Hydroxybutylmethacrylat-Copolymer;
   Resorcin, Glyoxal, AMPS/Hydroxybutylmethacrylat-Copolymer;
   Resorcin, Glyoxylsäure, AMPS/Hydroxybutylmethacrylat-Copolymer
Resorcin, Glutardialdehyd, AMPS/Hydroxybutylacrylat-Copolymer;
   Resorcin, Succindialaldehyd, AMPS/Hydroxybutylacrylat-Copolymer;
   Resorcin, Glyoxal, AMPS/Hydroxybutylacrylat-Copolymer;
   Resorcin, Glyoxylsäure, AMPS/Hydroxybutylacrylat-Copolymer
Resorcin, Glutardialdehyd, AMPS/Polyethylenglykolmonomethacrylat-Copolymer;
   Resorcin, Succindialaldehyd, AMPS/Polyethylenglykolmonomethacrylat-Copolymer;
   Resorcin, Glyoxal, AMPS/ Polyethylenglykolmonomethacrylat-Copolymer;
   Resorcin, Glyoxylsäure, AMPS/ Polyethylenglykolmonomethacrylat-Copolymer;
Resorcin, Glutardialdehyd, AMPS/Polyethylenglykolmonoacrylat-Copolymer;
   Resorcin, Succindialaldehyd, AMPS/Polyethylenglykolmonoacrylat-Copolymer;
   Resorcin, Glyoxal, AMPS/ Polyethylenglykolmonoacrylat-Copolymer;
   Resorcin, Glyoxylsäure, AMPS/ Polyethylenglykolmonoacrylat-Copolymer;
Resorcin, Glutardialdehyd, AMPS/Polypropylenglykolmonomethacrylat-Copolymer;
   Resorcin, Succindialaldehyd, AMPS/Polypropylenglykolmonomethacrylat-Copolymer;
   Resorcin, Glyoxal, AMPS/ Polypropylenglykolmonomethacrylat-Copolymer;
   Resorcin, Glyoxylsäure, AMPS/ Polypropylenglykolmonomethacrylat-Copolymer;
Resorcin, Glutardialdehyd, AMPS/Polypropylenglykolmonoacrylat-Copolymer;
   Resorcin, Succindialaldehyd, AMPS/Polypropylenglykolmonoacrylat-Copolymer;
   Resorcin, Glyoxal, AMPS/ Polypropylenglykolmonoacrylat-Copolymer;
   Resorcin, Glyoxylsäure, AMPS/ Polypropylenglykolmonoacrylat-Copolymer;
Resorcin, Glutardialdehyd, AMPS/Methoxy-polyethylenglykolmonomethacrylat-Copolymer;
   Resorcin, Succindialaldehyd, AMPS/Methoxy-polyethylenglykolmonomethacrylat-Copolymer;
   Resorcin, Glyoxal, AMPS/ Methoxy-polyethylenglykolmonomethacrylat-Copolymer;
   Resorcin, Glyoxylsäure, AMPS/ Methoxy-polyethylenglykolmonomethacrylat-Copolymer;
Resorcin, Glutardialdehyd, AMPS/Methoxy-polyethylenglykolmonoacrylat-Copolymer;
   Resorcin, Succindialaldehyd, AMPS/Methoxy-polyethylenglykolmonoacrylat-Copolymer;
   Resorcin, Glyoxal, AMPS/ Methoxy-polyethylenglykolmonoacrylat-Copolymer;
   Resorcin, Glyoxylsäure, AMPS/ Methoxy-polyethylenglykolmonoacrylat-Copolymer;
Harnstoff, Glutardialdehyd, AMPS/Hydroxyethylmethacrylat-Copolymer;
   Harnstoff, Succindialaldehyd, AMPS/Hydroxyethylmethacrylat-Copolymer;
   Harnstoff, Glyoxal, AMPS/Hydroxyethylmethacrylat-Copolymer;
   Harnstoff, Glyoxylsäure, AMPS/Hydroxyethylmethacrylat-Copolymer
Harnstoff, Glutardialdehyd, AMPS/Hydroxyethylacrylat-Copolymer;
   Harnstoff, Succindialaldehyd, AMPS/Hydroxyethylacrylat-Copolymer;
   Harnstoff, Glyoxal, AMPS/Hydroxyethylacrylat-Copolymer;
   Harnstoff, Glyoxylsäure, AMPS/Hydroxyethylacrylat-Copolymer
Harnstoff, Glutardialdehyd, AMPS/Hydroxypropylmethacrylat-Copolymer;
   Harnstoff, Succindialaldehyd, AMPS/Hydroxypropylmethacrylat-Copolymer;
   Harnstoff, Glyoxal, AMPS/Hydroxypropylmethacrylat-Copolymer;
   Harnstoff, Glyoxylsäure, AMPS/Hydroxypropylmethacrylat-Copolymer
Harnstoff, Glutardialdehyd, AMPS/Hydroxypropylacrylat-Copolymer;
   Harnstoff, Succindialaldehyd, AMPS/Hydroxypropylacrylat-Copolymer;
   Harnstoff, Glyoxal, AMPS/Hydroxypropylacrylat-Copolymer;
   Harnstoff, Glyoxylsäure, AMPS/Hydroxypropylacrylat-Copolymer
Harnstoff, Glutardialdehyd, AMPS/Hydroxybutylmethacrylat-Copolymer;
   Harnstoff, Succindialaldehyd, AMPS/Hydroxybutylmethacrylat-Copolymer;
   Harnstoff, Glyoxal, AMPS/Hydroxybutylmethacrylat-Copolymer;
   Harnstoff, Glyoxylsäure, AMPS/Hydroxybutylmethacrylat-Copolymer
Harnstoff, Glutardialdehyd, AMPS/Hydroxybutylacrylat-Copolymer;
   Harnstoff, Succindialaldehyd, AMPS/Hydroxybutylacrylat-Copolymer;
   Harnstoff, Glyoxal, AMPS/Hydroxybutylacrylat-Copolymer;
   Harnstoff, Glyoxylsäure, AMPS/Hydroxybutylacrylat-Copolymer
Harnstoff, Glutardialdehyd, AMPS/Polyethylenglykolmonomethacrylat-Copolymer;
   Harnstoff, Succindialaldehyd, AMPS/Polyethylenglykolmonomethacrylat-Copolymer;
   Harnstoff, Glyoxal, AMPS/ Polyethylenglykolmonomethacrylat-Copolymer;
   Harnstoff, Glyoxylsäure, AMPS/ Polyethylenglykolmonomethacrylat-Copolymer;
Harnstoff, Glutardialdehyd, AMPS/Polyethylenglykolmonoacrylat-Copolymer;
   Harnstoff, Succindialaldehyd, AMPS/Polyethylenglykolmonoacrylat-Copolymer;
   Harnstoff, Glyoxal, AMPS/ Polyethylenglykolmonoacrylat-Copolymer;
   Harnstoff, Glyoxylsäure, AMPS/ Polyethylenglykolmonoacrylat-Copolymer;
Harnstoff, Glutardialdehyd, AMPS/Polypropylenglykolmonomethacrylat-Copolymer;
   Harnstoff, Succindialaldehyd, AMPS/Polypropylenglykolmonomethacrylat-Copolymer;
   Harnstoff, Glyoxal, AMPS/ Polypropylenglykolmonomethacrylat-Copolymer;
   Harnstoff, Glyoxylsäure, AMPS/ Polypropylenglykolmonomethacrylat-Copolymer;
Harnstoff, Glutardialdehyd, AMPS/Polypropylenglykolmonoacrylat-Copolymer;
   Harnstoff, Succindialaldehyd, AMPS/Polypropylenglykolmonoacrylat-Copolymer;
   Harnstoff, Glyoxal, AMPS/ Polypropylenglykolmonoacrylat-Copolymer;
   Harnstoff, Glyoxylsäure, AMPS/ Polypropylenglykolmonoacrylat-Copolymer;
Harnstoff, Glutardialdehyd, AMPS/Methoxy-polyethylenglykolmonomethacrylat-Copolymer;
   Harnstoff, Succindialaldehyd, AMPS/Methoxy-polyethylenglykolmonomethacrylat-Copolymer;
   Harnstoff, Glyoxal, AMPS/ Methoxy-polyethylenglykolmonomethacrylat-Copolymer;
   Harnstoff, Glyoxylsäure, AMPS/ Methoxy-polyethylenglykolmonomethacrylat-Copolymer;
Harnstoff, Glutardialdehyd, AMPS/Methoxy-polyethylenglykolmonoacrylat-Copolymer;
   Harnstoff, Succindialaldehyd, AMPS/Methoxy-polyethylenglykolmonoacrylat-Copolymer;
   Harnstoff, Glyoxal, AMPS/ Methoxy-polyethylenglykolmonoacrylat-Copolymer;
   Harnstoff, Glyoxylsäure, AMPS/ Methoxy-polyethylenglykolmonoacrylat-Copolymer;
Melamin, Glutardialdehyd, AMPS/Hydroxyethylmethacrylat-Copolymer;
   Melamin, Succindialaldehyd, AMPS/Hydroxyethylmethacrylat-Copolymer;
   Melamin, Glyoxal, AMPS/Hydroxyethylmethacrylat-Copolymer;
   Melamin, Glyoxylsäure, AMPS/Hydroxyethylmethacrylat-Copolymer
Melamin, Glutardialdehyd, AMPS/Hydroxyethylacrylat-Copolymer;
   Melamin, Succindialaldehyd, AMPS/Hydroxyethylacrylat-Copolymer;
   Melamin, Glyoxal, AMPS/Hydroxyethylacrylat-Copolymer;
   Melamin, Glyoxylsäure, AMPS/Hydroxyethylacrylat-Copolymer
Melamin, Glutardialdehyd, AMPS/Hydroxypropylmethacrylat-Copolymer;
   Melamin, Succindialaldehyd, AMPS/Hydroxypropylmethacrylat-Copolymer;
   Melamin, Glyoxal, AMPS/Hydroxypropylmethacrylat-Copolymer;
   Melamin, Glyoxylsäure, AMPS/Hydroxypropylmethacrylat-Copolymer
Melamin, Glutardialdehyd, AMPS/Hydroxypropylacrylat-Copolymer;
   Melamin, Succindialaldehyd, AMPS/Hydroxypropylacrylat-Copolymer;
   Melamin, Glyoxal, AMPS/Hydroxypropylacrylat-Copolymer;
   Melamin, Glyoxylsäure, AMPS/Hydroxypropylacrylat-Copolymer
Melamin, Glutardialdehyd, AMPS/Hydroxybutylmethacrylat-Copolymer;
   Melamin, Succindialaldehyd, AMPS/Hydroxybutylmethacrylat-Copolymer;
   Melamin, Glyoxal, AMPS/Hydroxybutylmethacrylat-Copolymer;
   Melamin, Glyoxylsäure, AMPS/Hydroxybutylmethacrylat-Copolymer
Melamin, Glutardialdehyd, AMPS/Hydroxybutylacrylat-Copolymer;
   Melamin, Succindialaldehyd, AMPS/Hydroxybutylacrylat-Copolymer;
   Melamin, Glyoxal, AMPS/Hydroxybutylacrylat-Copolymer;
   Melamin, Glyoxylsäure, AMPS/Hydroxybutylacrylat-Copolymer
Melamin, Glutardialdehyd, AMPS/Polyethylenglykolmonomethacrylat-Copolymer;
   Melamin, Succindialaldehyd, AMPS/Polyethylenglykolmonomethacrylat-Copolymer;
   Melamin, Glyoxal, AMPS/ Polyethylenglykolmonomethacrylat-Copolymer;
   Melamin, Glyoxylsäure, AMPS/ Polyethylenglykolmonomethacrylat-Copolymer;
Melamin, Glutardialdehyd, AMPS/Polyethylenglykolmonoacrylat-Copolymer;
   Melamin, Succindialaldehyd, AMPS/Polyethylenglykolmonoacrylat-Copolymer;
   Melamin, Glyoxal, AMPS/ Polyethylenglykolmonoacrylat-Copolymer;
   Melamin, Glyoxylsäure, AMPS/ Polyethylenglykolmonoacrylat-Copolymer;
Melamin, Glutardialdehyd, AMPS/Polypropylenglykolmonomethacrylat-Copolymer;
   Melamin, Succindialaldehyd, AMPS/Polypropylenglykolmonomethacrylat-Copolymer;
   Melamin, Glyoxal, AMPS/ Polypropylenglykolmonomethacrylat-Copolymer;
   Melamin, Glyoxylsäure, AMPS/ Polypropylenglykolmonomethacrylat-Copolymer;
Melamin, Glutardialdehyd, AMPS/Polypropylenglykolmonoacrylat-Copolymer;
   Melamin, Succindialaldehyd, AMPS/Polypropylenglykolmonoacrylat-Copolymer;
   Melamin, Glyoxal, AMPS/ Polypropylenglykolmonoacrylat-Copolymer;
   Melamin, Glyoxylsäure, AMPS/ Polypropylenglykolmonoacrylat-Copolymer;
Melamin, Glutardialdehyd, AMPS/Methoxy-polyethylenglykolmonomethacrylat-Copolymer;
   Melamin, Succindialaldehyd, AMPS/Methoxy-polyethylenglykolmonomethacrylat-Copolymer;
   Melamin, Glyoxal, AMPS/ Methoxy-polyethylenglykolmonomethacrylat-Copolymer;
   Melamin, Glyoxylsäure, AMPS/ Methoxy-polyethylenglykolmonomethacrylat-Copolymer;
Melamin, Glutardialdehyd, AMPS/Methoxy-polyethylenglykolmonoacrylat-Copolymer;
   Melamin, Succindialaldehyd, AMPS/Methoxy-polyethylenglykolmonoacrylat-Copolymer;
   Melamin, Glyoxal, AMPS/ Methoxy-polyethylenglykolmonoacrylat-Copolymer;
   Melamin, Glyoxylsäure, AMPS/ Methoxy-polyethylenglykolmonoacrylat-Copolymer;

Ebenfalls für die erfindungsgemäßen Mikrokapseln geeignet sind Kombinationen, worin die vorgenannten Komponenten a) und b) und als Komponente c) anstelle von AMPS AMPP enthalten ist. Davon sind die folgenden AMPP-Kombinationen besonders bevorzugt:
Resorcin, Glutardialdehyd, AMPP/Polyethylenglykolmonomethacrylat-Copolymer;
   Resorcin, Succindialaldehyd, AMPP/Polyethylenglykolmonomethacrylat-Copolymer;
   Resorcin, Glyoxal, AMPP/ Polyethylenglykolmonomethacrylat-Copolymer;
   Resorcin, Glyoxylsäure, AMPS/ Polyethylenglykolmonomethacrylat-Copolymer;
Resorcin, Glutardialdehyd, AMPP/Polyethylenglykolmonoacrylat-Copolymer;
   Resorcin, Succindialaldehyd, AMPP/Polyethylenglykolmonoacrylat-Copolymer;
   Resorcin, Glyoxal, AMPP/ Polyethylenglykolmonoacrylat-Copolymer;
   Resorcin, Glyoxylsäure, AMPP/ Polyethylenglykolmonoacrylat-Copolymer;
Resorcin, Glutardialdehyd, AMPP/Polypropylenglykolmonomethacrylat-Copolymer;
   Resorcin, Succindialaldehyd, AMPP/Polypropylenglykolmonomethacrylat-Copolymer;
   Resorcin, Glyoxal, AMPP/ Polypropylenglykolmonomethacrylat-Copolymer;
   Resorcin, Glyoxylsäure, AMPP/ Polypropylenglykolmonomethacrylat-Copolymer;
Resorcin, Glutardialdehyd, AMPP/Polypropylenglykolmonoacrylat-Copolymer;
   Resorcin, Succindialaldehyd, AMPP/Polypropylenglykolmonoacrylat-Copolymer;
   Resorcin, Glyoxal, AMPP/ Polypropylenglykolmonoacrylat-Copolymer;
   Resorcin, Glyoxylsäure, AMPP/ Polypropylenglykolmonoacrylat-Copolymer;
Resorcin, Glutardialdehyd, AMPP/Methoxy-polyethylenglykolmonomethacrylat-Copolymer;
   Resorcin, Succindialaldehyd, AMPP/Methoxy-polyethylenglykolmonomethacrylat-Copolymer;
   Resorcin, Glyoxal, AMPP/ Methoxy-polyethylenglykolmonomethacrylat-Copolymer;
   Resorcin, Glyoxylsäure, AMPP/ Methoxy-polyethylenglykolmonomethacrylat-Copolymer;
Resorcin, Glutardialdehyd, AMPP/Methoxy-polyethylenglykolmonoacrylat-Copolymer;
   Resorcin, Succindialaldehyd, AMPP/Methoxy-polyethylenglykolmonoacrylat-Copolymer;
   Resorcin, Glyoxal, AMPP/ Methoxy-polyethylenglykolmonoacrylat-Copolymer;
   Resorcin, Glyoxylsäure, AMPS/ Methoxy-polyethylenglykolmonoacrylat-Copolymer;
Phloroglucin, Glutardialdehyd, AMPP/Polyethylenglykolmonomethacrylat-Copolymer;
   Phloroglucin, Succindialaldehyd, AMPP/Polyethylenglykolmonomethacrylat-Copolymer;
   Phloroglucin, Glyoxal, AMPP/ Polyethylenglykolmonomethacrylat-Copolymer;
   Phloroglucin, Glyoxylsäure, AMPS/Polyethylenglykolmonomethacrylat-Copolymer;
Phloroglucin, Glutardialdehyd, AMPP/Polyethylenglykolmonoacrylat-Copolymer;
   Phloroglucin, Succindialaldehyd, AMPP/Polyethylenglykolmonoacrylat-Copolymer;
   Phloroglucin, Glyoxal, AMPP/ Polyethylenglykolmonoacrylat-Copolymer;
   Phloroglucin, Glyoxylsäure, AMPP/ Polyethylenglykolmonoacrylat-Copolymer;
Phloroglucin, Glutardialdehyd, AMPP/Polypropylenglykolmonomethacrylat-Copolymer;
   Phloroglucin, Succindialaldehyd, AMPP/Polypropylenglykolmonomethacrylat-Copolymer;
   Phloroglucin, Glyoxal, AMPP/ Polypropylenglykolmonomethacrylat-Copolymer;
   Phloroglucin, Glyoxylsäure, AMPP/ Polypropylenglykolmonomethacrylat-Copolymer;
Phloroglucin, Glutardialdehyd, AMPP/Polypropylenglykolmonoacrylat-Copolymer;
   Phloroglucin, Succindialaldehyd, AMPP/Polypropylenglykolmonoacrylat-Copolymer;
   Phloroglucin, Glyoxal, AMPP/ Polypropylenglykolmonoacrylat-Copolymer;
   Phloroglucin, Glyoxylsäure, AMPP/ Polypropylenglykolmonoacrylat-Copolymer;
Phloroglucin, Glutardialdehyd, AMPP/Methoxy-polyethylenglykolmonomethacrylat-Copolymer;
   Phloroglucin, Succindialaldehyd, AMPP/Methoxy-polyethylenglykolmonomethacrylat-Copolymer;
   Phloroglucin, Glyoxal, AMPP/ Methoxy-polyethylenglykolmonomethacrylat-Copolymer;
   Phloroglucin, Glyoxylsäure, AMPP/ Methoxy-polyethylenglykolmonomethacrylatCopolymer;
Phloroglucin, Glutardialdehyd, AMPP/Methoxy-polyethylenglykolmonoacrylat-Copolymer;
   Phloroglucin, Succindialaldehyd, AMPP/Methoxy-polyethylenglykolmonoacrylat-Copolymer;
   Phloroglucin, Glyoxal, AMPP/ Methoxy-polyethylenglykolmonoacrylat-Copolymer;
   Phloroglucin, Glyoxylsäure, AMPP/ Methoxy-polyethylenglykolmonoacrylatCopolymer;

Für die erfindungsgemäßen Mikrokapseln sind auch Kombinationen mit zwei oder mehr Komponenten a) geeignet, insbesondere die vorgenannten Kombinationen, bei denen die Komponente a) ersetzt ist durch eine Kombination aus zwei oder mehr Verbindungen a), vorzugsweise durch zwei Amine a1) oder durch zwei aromatische oder heteroaromatische Verbindungen a2). Ebenso geeignet sind die vorgenannten Kombinationen, bei denen die Komponente a) aus einer Kombinationen von Komponenten a1) und a2) besteht. Beispiele solcher Kombinationen mit mehr als einer Komponente a) sind nachfolgend genannt:
Phloroglucin/Melamin, Glutardialdehyd, AMPS/ Methoxy-polyethylenglykolmonoacrylat-Copolymer;
Resorcin/Melamin, Glutardialdehyd, AMPS/ Methoxy-polyethylenglykolmonoacrylat-Copolymer;
Harnstoff/Melamin, Glutardialdehyd, AMPS/ Methoxy-polyethylenglykolmonoacrylat-Copolymer;
Harnstoff/Melamin, Glutardialdehyd, AMPP/ Methoxy-polyethylenglykolmonoacrylat-Copolymer.

Die Erfindung umfasst auch physikalische Mischungen verschiedener Mikrokapseln, so dass z.B. Kapselmischungen auf Basis der vorgenannten Mikrokapselsysteme in jedmöglicher Kombination umfasst sind. Dabei können die Eigenschaften je nach Anforderung durch die Art der gemischten Mikrokapseln und deren Mischungsverhältnis eingestellt werden.

Beispiel einer solchen Mischung von Mikrokapseln ist eine Kombination von Mikrokapseln aus Melamin, Glutardialdehyd, AMPS und PEM63PHD mit Mikrokapseln aus Phloroglucin, Glutardialdehyd, AMPS und PEM63PHD, z.B. in Mengenverhältnissen von 5:95 bis 95:5, wobei die Eigenschaften je nach Anforderung eingestellt werden können, z.B. bei Kernmaterial Duftöl eine höhere Lagerstabilität oder eine langzeitbeständige Freisetzung (Boost) der Duftstoffe.

In einer Ausführung der Erfindung können zur Herstellung der erfindungsgemäßen Mikrokapseln zusätzlich eines oder mehrere siliciumdioxidhaltige Agentien wie amorphes hydrophobes Kieselgel verwendet werden. Diese eignen sich insbesondere zur Nachbehandlung der Oberfläche der Mikrokapseln, z.B. um die Neigung zur Agglomeration zu reduzieren.

In einer Ausführung der Erfindung können zur Nachbehandlung der erfindungsgemäßen Mikrokapseln auch ein oder mehrere stickstoffhaltige oder sauerstoffhaltige Agentien verwendet werden. Als Beispiel für sauerstoffhaltige Agentien sind insbesondere Resorcin und Phloroglucin geeignet.
Von den stickstoffhaltigen Agentien kommen bevorzugt heterocyclische Verbindungen mit mindestens einem Stickstoffatom als Heteroatom, welches entweder mit einem aminosubstituierten Kohlenstoffatom oder einer Carbonylgruppe benachbart ist, wie zum Beispiel Pyridazin, Pyrimidin, Pyrazin, Pyrrolidon, Aminopyridin und davon abgeleitete Verbindungen zum Einsatz. Vorteilhafte Verbindungen dieser Gattung sind Aminotriazine und davon abgeleitete Verbindungen. Geeignet sind prinzipiell alle Aminotriazine, wie zum Beispiel Melamin, 2,6-Diaminotriazin, substituierte und dimere Aminotriazine und aus diesen Verbindungen hergestellte Mischungen. Vorteilhaft sind weiterhin Polyamide und Dicyandiamid, Harnstoff und seine Derivate sowie Pyrrolidon und davon abgeleitete Verbindungen. Beispiele für geeignete Pyrrolidone sind zum Beispiel Imidazolidinon und davon abgeleitete Verbindungen, wie zum Beispiel Hydantoin, dessen Derivate besonders vorteilhaft sind, insbesondere vorteilhaft sind von diesen Verbindungen Allantoin und seine Derivate. Besonders vorteilhaft sind weiter Triamino-1,3,5-Triazin (Melamin) und seine Derivate.
Es sei besonders betont, dass es sich bei der Nachbehandlung um eine "reine" Nachbehandlung der Oberfläche handelt, um zu dieser bevorzugten Ausführungsform der erfindungsgemäßen Mikrokapseln zu gelangen. Mit anderen Worten: in dieser bevorzugten Ausführungsform ist das eingesetzt stickstoffhaltige, sauerstoffhaltige oder siliziumhaltige Agens nicht gleichmäßig am Aufbau der gesamten Kapselwand beteiligt sondern überwiegend auf die äußere Oberfläche der Kapselwände konzentriert. Die zur Nachbehandlung eingesetzten Verbindungen werden bevorzugt als Schlemmen eingesetzt.

Ein weiterer Gegenstand der vorliegenden Offenbarung sind Mikrokapseldispersionen, die eine oder mehrere der erfindungsgemäßen Mikrokapseln enthalten.

Gegenstand der vorliegenden Offenbarung ist außerdem die Verwendung mindestens eines erfindungsgemäß umzusetzenden Amins a1) und/oder mindestens einer aromatischen oder heteroaromatischen Verbindung wie einem aromatischen Alkohol (oder dessen Derivat, insbesondere Ether) a2), zur Umsetzung mit einer erfindungsgemäß umzusetzenden aldehydischen Komponente b) zur Ausbildung der Kapselwände von Mikrokapseln. Dabei kann der freie Alkohol oder dessen Ether auch als Mischung vorliegen. Es ist bevorzugt, dass durch die offenbarte Verwendung formaldehydfreie Mikrokapseln zur Verfügung gestellt werden. Es können dem Reaktionsgemisch allerdings geringe Mengen Formaldehyd zugesetzt werden, im Allgemeinen weniger als 0,05 Gew.-%, vorzugsweise weniger als 0,01 Gew.-%, jeweils bezogen auf den Gesamtansatz, beispielsweise als Konservierungsmittel.

Der vorliegenden Erfindung liegt als weiterer Gegenstand ein Verfahren zur Herstellung der erfindungsgemäßen Mikrokapseln oder Mikrokapseldispersionen zu Grunde, wobei mindestens ein Amin und/oder aromatische oder heteroaromatische Verbindung wie ein aromatischer Alkohol, mindestens eine aldehydische Komponente, die mindestens zwei C-Atome pro Molekül aufweist, und in Gegenwart mindestens eines Copolymers, welches Einheiten von AMPS und/oder AMPP und einem oder mehreren (Meth)Acrylatmonmeren enthält, gegebenenfalls in Gegenwart mindestens einer zu verkapselnde Substanz (Kernmaterial), zusammengebracht und zur Reaktion gebracht werden und durch spätere Temperaturerhöhung die Aushärtung der Kapseln erfolgt. Dabei ist es besonders bevorzugt, dass der pH-Wert im Laufe des Verfahrens erhöht wird, insbesondere vor der Aushärtung.

Vorzugweise wird im Rahmen des erfindungsgemäßen Verfahrens zunächst
a) mindestens ein Amin a1) und/oder mindestens eine aromatische oder heteroaromatische Verbindung wie ein aromatischer Alkohol und/oder dessen Derivat (z.B. Ester) oder Ether a2), und mindestens eine aldehydische Komponente b), in Gegenwart mindestens eines Copolymers c), welches Einheiten von AMPS und/oder AMPP und einem oder mehreren (Meth)Acrylatmonomeren enthält, und mindestens eine zu verkapselnde Substanz bei einer Temperatur von 40 bis 65°C und einem pH-Wert zwischen 6 und 9, vorzugsweise zwischen 7 und 8,5 zusammengebracht und
b) in einem späteren Verfahrensschritt bei einer Temperatur von 40 bis 65 °C der pH-Wert zwischen 2 und 11 eingestellt, vorzugsweise im Fall von Resorcin auf über 9, bevorzugt zwischen 9,5 und 11, im Fall von Phloroglucin auf kleiner 4, bevorzugt zwischen 3 und 4, und im Fall von Melamin und Harnstoff im Bereich von 2 bis 7, bevorzugt zwischen 3 und 6,
c) wobei später die Aushärtung der Kapseln durch Temperaturerhöhung auf 40°C bis 110°C, vorzugsweise 70°C bis 90°C, insbesondere 80 °C durchgeführt wird.

Durch die gewählten Parameter der Zudosiergeschwindigkeit, der Temperatur, des pH-Wertes und/oder der Rührgeschwindigkeit können die Ausbeute und Qualität der erfindungsgemäßen Mikrokapseln oder Mikrokapseldispersionen beeinflusst werden. Insbesondere kann eine zu niedrige Temperatur dazu führen, dass die Kapselwand weniger dicht ausgebildet wird. Dies ist dem Fachmann erkennbar an einer bleibenden Ölphase, verminderten Ausbeute sowie Abscheidung von Kernmaterial als Kondensat im Filter des Trockners. Andererseits sollte darauf geachtet werden, dass die Reaktionsgeschwindigkeit nicht zu hoch ist, da sich sonst nur wenig Wandmaterial um die Kapseln legt, bzw. zu viel freies Wandmaterial außerhalb der Kapseln vorliegt. Dieses freie Wandmaterial kann dann in Teilchen vorliegen, die größer sind als die Kapseln.

Die Alkalität kann ebenfalls von Bedeutung für die Qualität der erfindungsgemäßen Mikrokapseln sein. Daneben beeinflusst der pH-Wert im Rahmen der Prozessführung die Tendenz zum Gelieren des Ansatzes.

In einer Ausführungsform des erfindungsgemäßen Verfahrens wird zum Einstellen der Alkalität ein Alkalisalz, vorzugsweise Alkalicarbonat, insbesondere Natriumcarbonat verwendet. Natriumcarbonat ist bevorzugt, da damit die Gefahr des Gelierens verringert wird. Es eignen sich für bestimmte Anforderungsprofile, z.B. für im saurenMedium besonders beständige Kapselsysteme insbesondere Alkalilaugen der 1. und 2. Hauptgruppe des Periodensystems der Elemente.

Im Rahmen des erfindungsgemäßen Verfahrens kann zu Beginn der Umsetzung (Verfahrensschritt a)) des Amins und/oder aromatischen Alkohols mit der aldehydischen Komponente gerührt werden, wobei die Rührgeschwindigkeit bei 500 bis 2500 U/min, insbesondere bei 1000 bis 2000 U/min liegen kann. Zu dem erhaltenen Gemisch kann anschließend mindestens ein Copolymer, welches Einheiten von AMPS und/oder AMPP und einem oder mehreren (Meth)Acrylatmonomeren enthält und die zu verkapselnde Substanz zugeben werden. Vorzugsweise wird später und zwar unmittelbar vor oder während der Anhebung der Alkalität (Verfahrensschritt b) die Rührgeschwindigkeit erhöht, wobei sie dann bei 3000 bis 5000 U/min, insbesondere bei 3500 bis 4500 U/min, vor allem bei 4000 U/min liegen kann.

Vorzugsweise wird die so erhöhte Rührgeschwindigkeit so lange beibehalten, bis die Viskositätswerte der Mischung sinken, wobei nach Einsetzen einer Viskositätsabnahme die Rührgeschwindigkeit gesenkt wird, vorzugsweise auf 500 bis 2500 U/min, besonders bevorzugt auf 1000 bis 2000 U/min. Ein früheres Absenken der Rührgeschwindigkeit kann ebenfalls zum unerwünschten Gelieren des Ansatzes führen.

Vorzugsweise wird nach Beginn der beschriebenen Viskositätsabnahme mindestens 20 Minuten, besonders bevorzugt zwischen 30 und 180 Minuten, vorzugsweise bei einer Rührgeschwindigkeit von 1000 bis 2000 U/min und einer Temperatur von 40 bis 65°C weiter gerührt, bevor in Verfahrensschritt c) die Aushärtung der Kapseln durch Temperaturerhöhung erfolgt. Diese Phase nach Beginn der beschriebenen Viskositätsabnahme und vor Aushärtung der Kapseln wird in der vorliegenden Erfindung auch als Ruhephase bezeichnet. Die Ruhephase kann vorzugsweise dazu dienen, um die Präformierung ausreichend stabiler Kapselwände zu erreichen, mit anderen Worten, die Kapselwände so stabil auszubilden, dass kein Kernmaterial mehr entweicht.

Die erfindungsgemäßen Mikrokapseln sind formaldehydfrei. Sie lassen sich als stabile Kern/Schale-Mikrokapseln aus der wässrigen Slurry heraus zu einem trockenen fließfähigen Pulver verarbeiten.
Die Beladung der Miokrokapseln kann mit hydrophoben und hydrophilen Materialien, mit gasförmigen, flüssigen und festen Substanzen erfolgen.

Ein weiterer Gegenstand der vorliegenden Offenbarung ist die Verwendung von erfindungsgemäßen Mikrokapseln oder Mikrokapseldispersionen für die kontrollierte Freisetzung von Kernmaterialen, die hydrophil (z.B. Aromastoffe) oder hydrophob sein können. Die Kernmaterialien sind beispielsweise Wirkstoffe, die vorzugsweise ausgewählt sind aus der Gruppe der Duft-und Aromastoffe, Pestizide, Herbizide, Schmiermittel, Gleitmittel (z.B. fluorierte Kohlenwasserstoffe), Insektizide, antimikrobiellen Wirkstoffe, pharmazeutischen Wirkstoffe, kosmetischen Wirkstoffe (z.B. für Shampoo), Latentwärmespeicher (z.B. Wachse), Katalysatoren (z. B. organische Carbonate), Selbstheilungsagenzien (z.B. Norbornen, Dicyclopentadien), Beschichtungssysteme wie Lacke (z.B. Duftlacke), Farbstoffe (z.B. für kohlefreie Durchschreibesysteme), hydrophobe Wachse, hydrophobe En-Komponenten oder hydrophobe Lösungsmittel.

Außerdem sind Gegenstand der vorliegenden Offenbarung Produkte, die erfindungsgemäße Mikrokapseln oder Mikrokapseldispersionen enthalten und deren Verwendung vorzugsweise auf einem Anwendungsfeld liegt, das ausgewählt ist aus den Gebieten der Beschichtungen, wie kohlefreie Durchschreibesysteme, Beschichtung und Imprägnierung von Papieren und Sicherheitsmerkmalbeschichtung, der katalysatorgefüllten Mikrokapseln, Lacktechnologie wie Lackherstellung, Bauchemie, Dentaltechnik vorzugsweise als Bestandteil für schnell härtende Zahnfüllmassen, Selbstheilungssysteme, Kosmetik vorzugsweise für Duft- und Aromaöle, Pharmazie vorzugsweise als Wirkstoffträger, Medizintechnik, z.B. zur Verkapselung von Emittenten von Neurotransmittern wie NO, z.B. von Nitroglycerin, Waschen, Reinigen, Desinfizieren, Kleben, Flammschutz, der Behandlung von Pflanzen, vorzugsweise als Fungizid, Pestizid, Insektizid, Herbizid oder Korrosionsschutz.

Die erfindungsgemäßen Mikrokapseln lassen sich z.B. für die Herstellung von Lacken z.B. für Duftlacke einsetzen und sind in Ihrem Vernetzungsgrad, ihrer Größe ihrer Wandstärke und Oberflächenausrüstung sowie im Kernmaterial variabel einsetzbar.

Aufgrund der hohen chemischen und physikalischen Resistenz eignen Sie sich als stabile Kern/Schale Kapselsysteme, auch für den Einsatz in aggressiven Medien. So ist es möglich Duftlacke herzustellen, die über herkömmliche Rakelsysteme mit den in der Druckindustrie bekannten Schichtstärken zu beschichten, ohne dass ein nennenswerter Anteil der Kapseln zerstört wird.

Die Mikrokapseln weisen im Allgemeinen einen mittleren Durchmesser von 1-1000 µm auf. Im Rahmen der vorliegenden Erfindung sind vom Begriff Mikrokapsel auch Nanokapseln umfasst, d.h. Kapseln mit einem mittleren Durchmesser < 1 µm. Die Kapseln weisen vorzugsweise einen mittleren Durchmesser von 0,1 bis 100 µm auf. Die Wandstärke ist einstellbar und kann 0,01-100 µm, insbesondere zum Beispiel 0,1 bis 10 µm betragen.

Es ist auch die Herstellung von Vollkugeln möglich, also Partikel, die kein Kernmaterial umschließen. Diese Vollkugeln können einen mittleren Durchmesser von unter 500nm (vorzugsweise zwischen 300 und 400 nm) aufweisen. Dabei kann es sich vorzugsweise um monodisperse Vollkugeln handeln. Zur Herstellung dieser Vollkugeln kann in einer Ausführungsform Phloroglucin verwendet werden.

Die erfindungsgemäßen Vollkugeln können als Standard- oder Kontrollansatz zum Beispiel in der Medizintechnik (z.B. als Eichlösung in Partikelsizern oder Erythrozytenzählern) Verwendung finden oder als abrasiver Bestandteil in Scheuermitteln, für dekorative Effekte oder als Abstandhalter für verdruckbare Lacke mit druckempfindlichen Partikeln genutzt werden.

Die erfindungsgemäßen Mikrokapseln können in Form wässriger Dispersionen als Tränkharze im Holz/Werkstoff-Bereich eingesetzt werden und eignen sich insbesondere als Imprägnierharze mit zusätzlichen Funktionen wie katalytische Effekte, Farbeffekte, thermochrome Effekte oder Sicherheitseffekte für dekorative Beschichtungssysteme.

Nachfolgend wird die vorliegende Erfindung anhand einiger Ausführungsbeispiele erläutert, diese haben rein illustrativen Charakter und beschränken die Erfindung in keiner Weise:

### Beispiel 1: Herstellung von Copolymeren

### a) AMPS-Hydroxybutylacrylat

Für den 1500 g Ansatz werden 891 g demineralisiertes Wasser zusammen mit 585 g AMPS (50-% wässrige Lösung) und 7,5 g 4-Hydroxybutylacrylat (HBA) in den Reaktor gefüllt und unter Schutzgas-Atmosphäre gesetzt. Das Reaktionsgemisch wird unter Rühren (400 U/min) auf 75 °C aufgeheizt. Von dem wasserlöslichen Initiator Natriumperoxodisulfat werden 0,03 g in 15 g Wasser gelöst und mittels einer Spritze in den Reaktor injiziert, wenn die Reaktionstemperatur erreicht ist. Nach Erreichen der Maximaltemperatur beginnt eine Stunde Nachreaktion. Anschließend wird der Ansatz auf Raumtemperatur abgekühlt und mit 1,5 g Konservierungsmittel versetzt.

Die wässrige Lösung wird charakterisiert über die Viskosität, Festkörper-Gehalt und den pH-Wert. Die Viskosität beträgt 540 mPas (gemessen bei 20 rpm Brookfield), der Festkörper-Gehalt beträgt 21 % und der pH-Wert liegt bei 3,3. Es werden 3 g Copolymer auf eine Petrischale gegeben und 24 Stunden bei 160 °C im Trockenschrank getrocknet. Die Auswaage beträgt 0,69 g, was einer Ausbeute von 21,6 % entspricht.

### b) AMPS-Polyakylenglykolmonomethacrylat

Die Vorlage besteht aus 912 g demineralisiertem Wasser, 240 g AMPS und 7,5 g Poly(ethylen/propylen)glykolmonomethacrylat (Bisomer PEM63PHD von Cognis, CAS-Nr. 589-75-9). Die Mischung wird unter Schutzgas-Atmosphäre gesetzt. Das Reaktionsgemisch wird unter Rühren (400 U/min) auf 75 °C aufgeheizt. 1,5 g Natriumperoxodisulfat werden in 15 g Wasser gelöst und mit einer Spritze in den Reaktor überführt. Nachdem die Temperatur im Reaktor ein Maximum erreicht hat und zu sinken beginnt, werden 240 g AMPS mit 83 g PEM63PHD mittels Schlauchpumpe über einen Zeitraum von einer Stunde zu dosiert. Anschließend erfolgt eine halbstündige Nachreaktion. Anschließend wird der Ansatz auf Raumtemperatur abgekühlt und mit 1,5 g Konservierungsmittel versetzt.

Die wässrige Lösung wird charakterisiert über die Viskosität, Festkörper-Gehalt und den pH-Wert. Die Viskosität beträgt 110 mPas (gemessen bei 20 rpm Brookfield), der Festkörper-Gehalt beträgt 23 % und der pH-Wert ist 3,1. Es werden 3 g Copolymer auf eine Petrischale gegeben und 24 Stunden bei 160 °C im Trockenschrank getrocknet. Die Auswaage beträgt 0,68 g, was einer Ausbeute von 21,6 % entspricht.

### Beispiel 2: Phloroglucin-Melamin Mikrokapsel

### a) Herstellung des Vorkondensats

5,4 g Phloroglucin und 0,6 g Melamin werden in 78,6 g destilliertem Wasser dispergiert. Der pH-Wert wird mit 1,2 g 85%iger Ameisensäure auf 3 eingestellt. Es wird auf 35 °C erwärmt und 14,2 g 50%ige Glutaraldehydlösung zugegeben. Nach 5 min beginnt sich das lösliche Vorkondensat zu bilden, erkennbar daran, dass das in Wasser kaum lösliche Phloroglucin und Melamin sich auflöst. Der Gesamtfestkörper des Vorkondensats beträgt 14,0 Gew.-%

### b) Herstellung der Mikrokapsel

41,5 g des in Stufe a) erhaltenen löslichen Vorkondensats werden nach 5 min mit 3,0 g des Schutzkolloids, einem Copolymer aus AMPS (2-Acrylamido-2-methyl-1-propylsulfonsäure und PEM 6 (Polyethylenglykolmonomethacrylat) und 23,7 g eines zu verkapselnden Duftöls versetzt. Zur Teilchenbildung wird gleichzeitig die Drehzahl von 500 U/min auf 2500 U/min erhöht. Nach 20 min beginnt das Harz zu strukturierten Kapselwänden auszuhärten. In der folgenden Stunde wird bei einer Umdrehungszahl von 600 U/min gerührt. Innerhalb dieser Stunde wird nach 15 min 7,5 g einer auf pH 3 mit Ameisensäure (85 %) angesäuerten 14 gew.-%igen Phloroglucin-Schlämme 45 min lang hinzudosiert, sowie nach 20 min 16 g Wasser hinzugegeben, um ein Dickwerden der Slurry zu vermeiden. Darauf folgt eine 2-stündige Aushärtungphase bei 80 °C. Anschließend wird 4,2 g einer mit Ameisensäure 85 % angesäuerten, 33 gew.%igen Melamin-Schlämme (Folco-Schlämme) eine ½ h zudosiert. Es wird schließlich ½ h bei pH- 3 nachgehärtet. Die Kapselslurry wird auf Raumtemperatur abgekühlt und mit Natronlauge auf pH 7 eingestellt.

Technische Daten der erhaltenen Mikrokapsel:

| | |
|---|---|
| Durchmesser D(90): | 10µm |
| Festkörper: | 33 % |
| Kernanteil: | 70 % |
| Effizienz: | 90 % |
| Pulverausbeute: | 90% |
| Restaldehydgehalt: | < 500 ppm, ermittelt mit GC (FT-IR) |

### Beispiel 3: Melamin Mikrokapsel

31,0 g Glutaraldehyd-Lösung (50%) werden mit 90g destilliertem Wasser auf 55°C erwärmt und mit Natronlauge (10%) auf pH 9,2 eingestellt. Anschließend werden 5,6g Melamin zugegeben und dieses Gemisch wird 10 Minuten bei 55°C vorkondensiert. Bei weiterhin 55°C werden 9,5g Copolymer aus AMPS (2-Acrylamido-2-methyl-1-propansulfonsäure) und PEM 6 (Polyethylenglycolmonomethacrylat) und des weiteren 78g Diethylphthalat zugegeben und die Drehzahl auf 1600 U/min erhöht. Nun wird mit Amidosulfonsäure (15%) der pH-Wert auf pH 6,1 erniedrigt. Nach ca. 2 Minuten stellt sich eine stabile Kapselgröße von ca. 30µm ein. Bei niedrigerer Drehzahl (800 U/min) werden die Kapseln 1h bei 55°C und 3h bei 80°C ausgehärtet. Während der Aushärtung werden dem Ansatz 4g Melamin zugegeben und die letzte Stunde der Aushärtung wird der pH-Wert mit Natronlauge (20%) bei pH 9-11 gehalten.

Technische Daten der erhaltenen Mikrokapsel:

| | |
|---|---|
| Durchmesser D(90): | 28µm |
| Festkörper: | 46,4% |
| Kernanteil: | 74,8% |
| Effizienz: | 97% |
| Pulverausbeute: | 50% |
| Restaldehydgehalt: | < 500ppm, ermittelt mit GC (FT-IR) |

### Beispiel 4: Allgemeine Synthesevorschrift für Terpolymere

Es werden 495,0 g Monomer 1 (AMPS oder AMPP) und 7,5 g Monomer 2 (Meth/Acrylatmonomer) und 898 g demineralisiertes Wasser als Vorlage in einen Doppelmantel-Glasreaktor überführt. Die Vorlage wird kurz durchmischt und dann 10 Minuten mit Argon begast. Anschließend wird das Reaktionsgemisch auf 75 °C erhitzt und mit einer Initiatorlösung (0,75 g in 15 g Wasser) gestartet. Nach dem Erreichen der maximalen Temperatur im Reaktor werden Monomer 2 (37,5 g) und Monomer 3 (Drittmonomer) (15 g) innerhalb einer Stunde mittels Schlauchpumpe oder Spritzenpumpe zudosiert. Nach der Zudosierung erfolgt eine Stunde Nachreaktion und anschließend wird die erhaltene wässrige Polymerlösung abgekühlt. Wenn die Temperatur im Reaktor unterhalb von 40 °C liegt werden 1,5 g Konservierer zugesetzt und das homogene Produkt abgefüllt.

### Beispiel 5: AMPS-Polyalkylenglykolmonomethacrylat-N-Vinylcaprolactam

Die Vorlage besteht aus 883 g demineralisiertem Wasser, 525 g AMPS, 7,5 g PEM 63 PHD und 15 g N-Vinylcaprolactam. Die Mischung wird unter Schutzgas-Atmosphäre gesetzt und unter Rühren (250 rpm) auf 75 °C aufgeheizt. 0,75 g Natriumperoxodisulfat werden in 15 g Wasser gelöst und mit einer Spritze in den Reaktor injiziert. Nachdem die Temperatur ein Maximum erreicht hat und zu sinken beginnt, werden 15 g N-Vinylcaprolactam mit 37,5 g PEM 63 PHD mittels Spritzenpumpe über einen Zeitraum von einer Stunde zu dosiert. Anschließend erfolgt eine halbstündige Nachreaktion. Danach wird der Ansatz auf Raumtemperatur abgekühlt und mit 1,5 g Konservierer versetzt.

Die wässrige Lösung wird charakterisiert über die Viskosität, Festkörper-Gehalt und den pH-Wert. Die Viskosität beträgt 1800 mPas (gemessen bei 20 rpm Brookfield), der Festkörper-Gehalt beträgt 22,1 % und der pH-Wert ist 3,5. Es werden 3 g Terpolymer auf eine Petrischale gegeben und 24 Stunden bei 160 °C im Trockenschrank getrocknet. Die Auswaage beträgt 0,67 g und entspricht einer Ausbeute 22,3 %.

## Patentansprüche

1. Mikrokapseln, deren Kapselwände ein Harz umfassen, welches durch Umsetzung
a) mindestens einer Verbindung ausgewählt aus der Gruppe der
a1) Amine und
a2) aromatischen oder heteroaromatischen Verbindungen, welche unsubstituiert oder mit einem oder mehreren Substituenten aus Gruppe C₁-C₂₀-Alkyl, OH, OR, COOH, SH, SR, NHCOR, OCOR, Halogen, Aromat substituiert sind, wobei R eine C₁₋C₁₀-Alkyl Gruppe darstellt,
mit
b) mindestens einer aldehydischen Komponente, die mindestens zwei C-Atome pro Molekül aufweist, in Gegenwart
c) mindestens eines Copolymeren, welches Einheiten von 2-Acrylamido-2-methyl-propansulfonsäure oder dessen Salzen (AMPS) und/oder 2-Acrylamido-2-methyl-propanphosphonsäure oder dessen Salzen (AMPP) und Einheiten von einem oder mehreren (Meth)Acrylaten enthält,
erhältlich ist, wobei auf die Verwendung von Formaldehyd verzichtet wird

2. Mikrokapseln nach Anspruch 1, worin als Komponente a) ein oder mehrere Verbindungen aus der Gruppe der Harnstoffe, Melamine und Benzoguanamine ausgewählt sind.

3. Mikrokapseln nach Anspruch 1, worin als Komponente a) ein oder mehrere aromatische oder heteroaromatische Alkohole und/oder ein oder mehrere aromatische oder heteroaromatische Carbonsäuren ausgewählt sind.

4. Mikrokapseln nach Anspruch 1 oder 3, worin als Komponente a) ein aromatischer Alkohol oder dessen Ether oder Derivat ausgewählt ist.

5. Mikrokapseln nach einem der Ansprüche 3 oder 4, worin der mindestens eine aromatische Alkohol ausgewählt ist aus den Phenolen mit zwei oder mehr Hydroxygruppen, vorzugsweise aus Brenzcatechin, Resorcin, Hydrochinon und 1,4-Naphthohydrochinon, Phloroglucin, Pyrogallol, Hydroxyhydrochinon, weiter vorzugsweise Resorcin und Phloroglucin.

6. Mikrokapseln nach einem der Ansprüche 3 bis 5, worin die Alkohole in Form ihrer Salze, Ether oder Ester und die Carbonsäuren in Form ihrer Salze oder Ester vorliegen.

7. Mikrokapseln nach Anspruch 1, worin die Komponente a) eine Mischung aus a1) einem oder mehreren Aminen, definiert wie in einem der Ansprüche 1 oder 2, und a2) einer oder mehreren aromatischen oder heteroaromatischen Verbindungen, definiert wie in einem der Ansprüche 1 und 3 bis 6, enthält.

8. Mikrokapseln nach einem der vorhergehenden Ansprüche, worin die aldehydische Komponente b) ausgewählt ist aus den aliphatischen und aromatischen Aldehyden.

9. Mikrokapseln nach einem der vorhergehenden Ansprüche, worin die aldehydische Komponente ausgewählt ist aus Valeraldehyd, Capronaldehyd, Caprylaldehyd, Decanal, Succindialdehyd, Cyclohexancarbaldehyd, Cyclopentancarbaldehyd, 2-Methyl-1-propanal , 2-Methylpropionaldehyd, Acetaldehyd, Acrolein, Aldosteron, Antimycin A, 8'-Apo-β-caroten-8'-al, Benzaldehyd, Butanal, Chloral, Citral, Citronellal, Crotonaldehyd, Dimethylaminobenzaldehyd, Folinsäure, Fosmidomycin, Furfural, Glutaraldehyd, Glycerinaldehyd, Glykolaldehyd, Glyoxal, Glyoxylsäure, Heptanal, 2-Hydroxybenzaldehyd, 3-Hydroxybutanal, Hydroxymethylfurfural, 4-Hydroxynonenal, Isobutanal, Isobutyraldehyd, Methacrolein, 2-Methylundecanal, Mucochlorsäure, N-Methylformamid, 2-Nitrobenzaldehyd, Nonanal, Octanal, Oleocanthal, Orlistat, Pentanal, Phenylethanal, Phycocyanin, Piperonal, Propanal, Propenal, Protocatechualdehyd, Retinal, Salicylaldehyd, Secologanin, Streptomycin, Strophanthidin, Tylosin, Vanillin, Zimtaldehyd.

10. Mikrokapseln nach einem der vorhergehenden Ansprüche, worin die aldehydische Komponente ausgewählt ist aus Glutardialdehyd und/oder Succindialdehyd.

11. Mikrokapseln nach einem der vorhergehenden Ansprüche, worin das Copolymer ein Bipolymer oder ein Terpolymer ist.

12. Mikrokapseln nach einem der vorhergehenden Ansprüche, worin das Copolymer c) aus Einheiten von AMPS oder AMPP, bevorzugt AMPS, und einem oder mehreren (Meth)Acrylaten aufgebaut ist.

13. Mikrokapseln nach Anspruch 1 oder 12, worin in Copolymer c) die (Meth)Acrylate ausgewählt sind aus der Gruppe
a) 4-Hydroxybutylacrylat 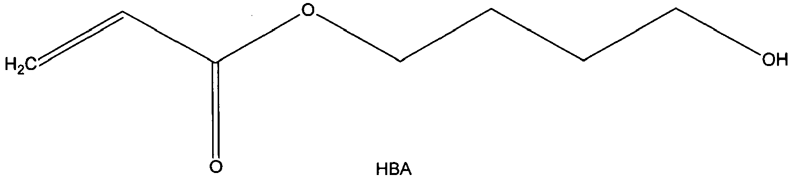
b) 2-Hydroxypropylmethacrylat 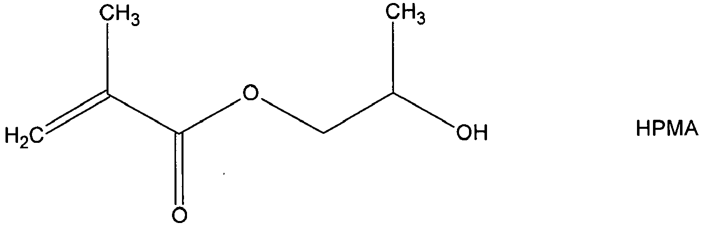
c) Ammoniumsulfatoethylmethacrylat 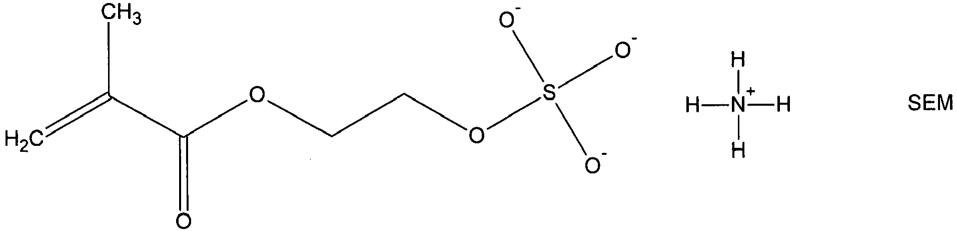
d) Pentapropylenglykolmethacrylat 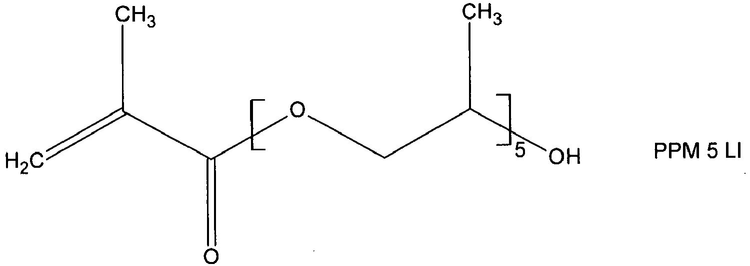
e) Acrylsäure 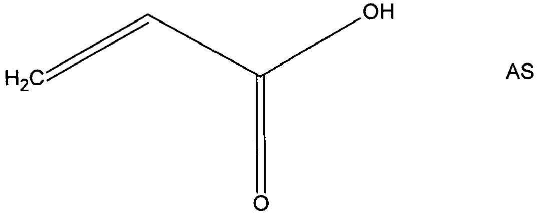
f) Hexaethylenglykolmethyacrylat 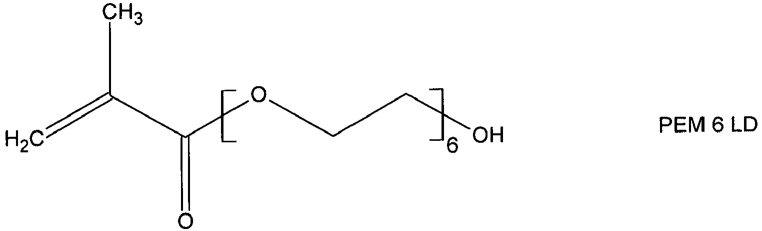
g) Hexapropylenglykolacrylat 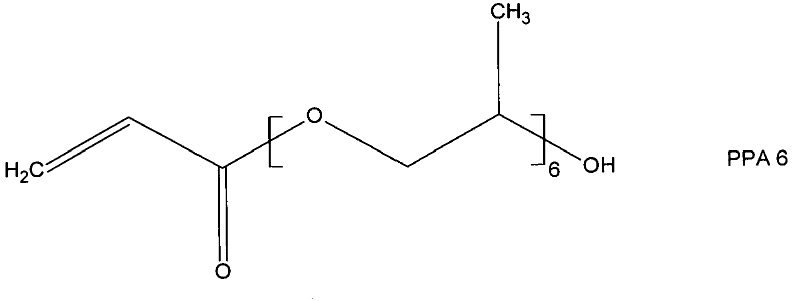
h) Hexaethylenglykolacrylat 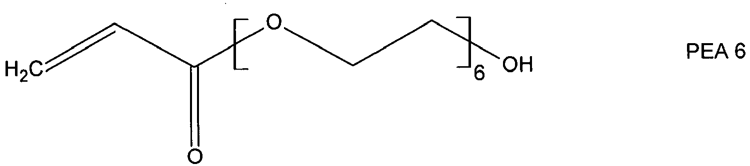
i) Hydroxyethylmethacrylat 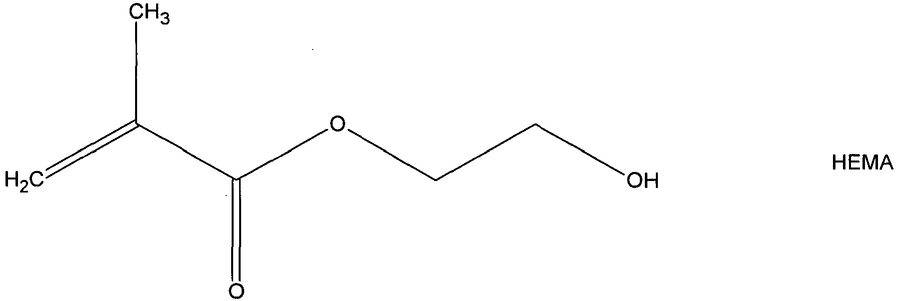
j) Polyalkylenglycolmethacrylat (CAS-Nr. 589-75-9) 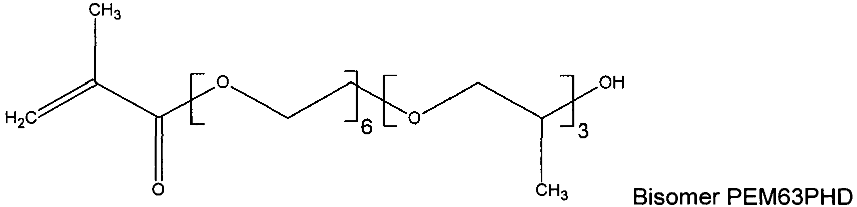
k) Methoxy-polyethylenglycolmethacrylat 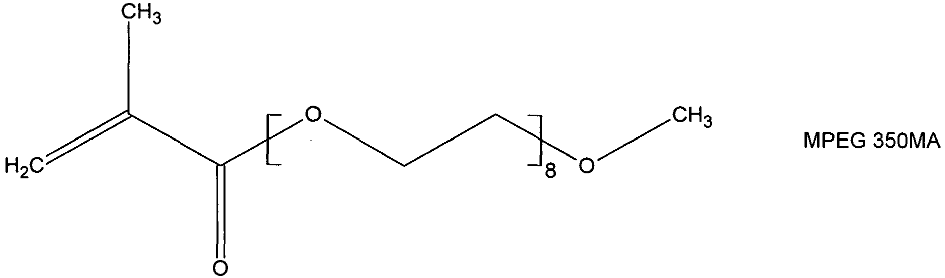
I) 2-Propylheptylacrylat (2-PHA) 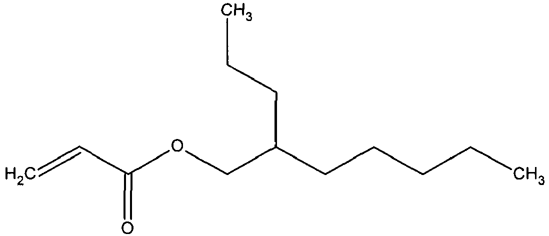
m) 1,3-Butandioldimethacrylat (BDDMA) 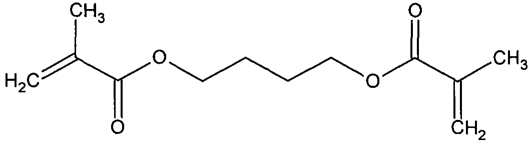
n) Triethylenglykoldimethacrylat (TEGDMA) 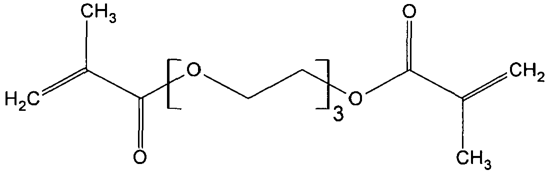
o) Hydroxyethylacrylat (HEA) 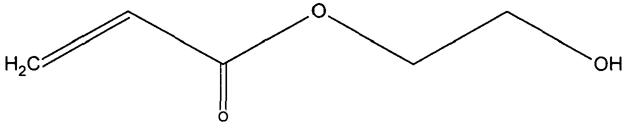
p) 2-Hydroxypropylacrylat (HPA) 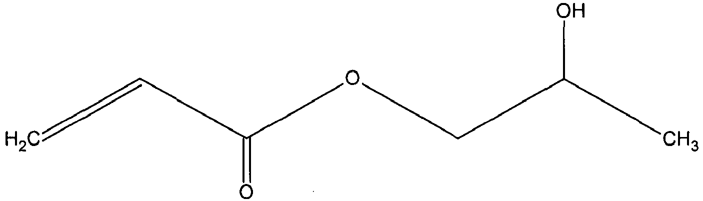
q) Ethylenglykoldimethacrylat (EGDMA) 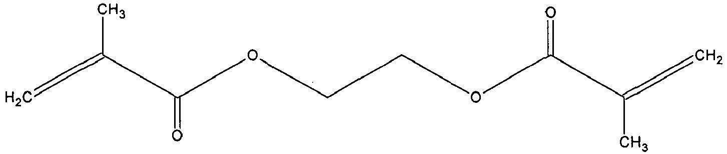
r) Glycidylmethacrylat (GMA) 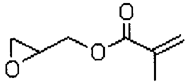
s) Allylmethacrylat (ALMA) 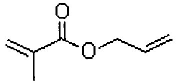
t) Bisomer PEM 3 (Polyethylenglykolmethacrylat) 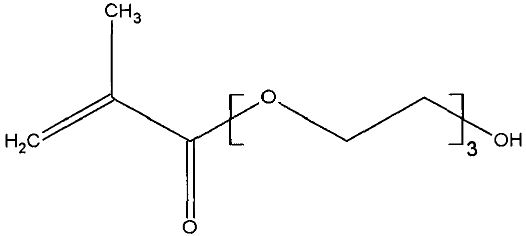
u) Bisomer IDMA (Isodecylmetacrylat) 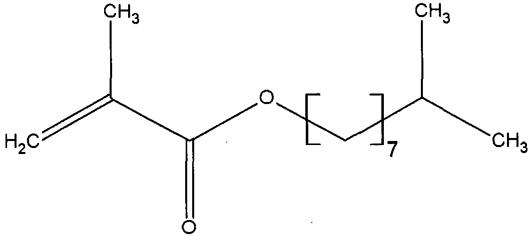
v) Bisomer C13MA (Isotridecylmethacrylat) 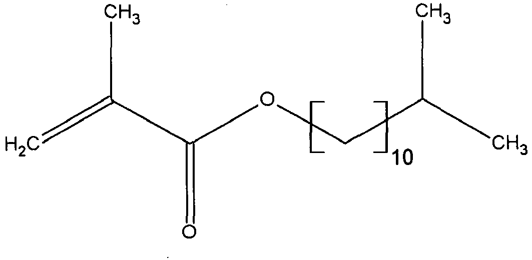 w) (3-Sulfopropyl)-acrylat
x) N-(2-Acryloyloxyethyl)-N,N-dimethyl-N-(3-sulfopropyl)ammoniumbetain 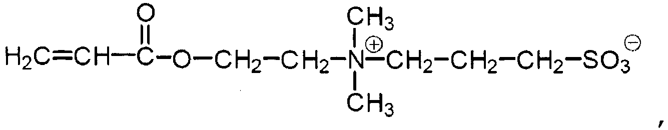
y) N-(2-Methacryloyloxyethyl)-N,N-dimethyl-N-(3-sulfopropyl)ammoniumbetain 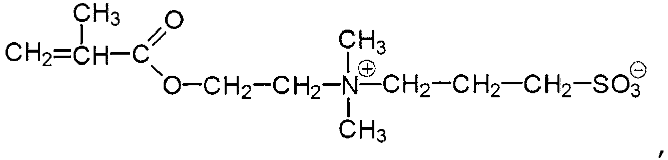
z) (3-Sulfopropyl)-methacrylat,
z1) N-(3-Methacrylamidopropyl)-N,N-dimethyl-N-(3-sulfopropyl)ammoniumbetain

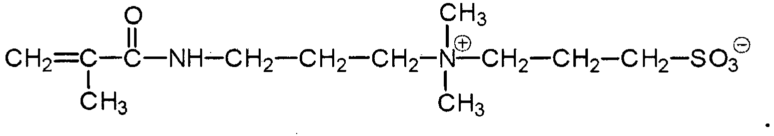

14. Mikrokapseln nach einem der vorhergehenden Ansprüche, worin das Copolymer c) ein oder mehrere weitere Monomere enthält, ausgewählt aus der Gruppe der Vinylverbindungen, der ungesättigten Di- oder Polycarbonsäuren und der Salze von Amylverbindungen oder Allylverbindungen.

15. Mikrokapseln nach einem der vorhergehenden Ansprüche, worin das molare Verhältnis der mindestens einen Komponente a), zu der mindestens einen aldehydischen Komponente b) zwischen 1:1 und 1:5, vorzugsweise zwischen 1:1 und 1:3 liegt.

16. Mikrokapseln nach einem der vorhergehenden Ansprüche, worin die Kapseloberfläche mit einem stickstoffhaltigen, sauerstoffhaltigen oder siliziumhaltigen Agens, vorzugsweise mit Melamin, mit Kieselgel oder aromatischem Alkohol a) nachbehandelt wird.

17. Verfahren zur Herstellung von Mikrokapseln gemäß einem oder mehreren der Ansprüche 1 bis 16, worin
a) eines oder mehrerer Amine a1), definiert wie in einem der Ansprüche 1 bis 2, und/oder eine oder mehrere aromatische oder heteroaromatische Verbindungen a2), definiert wie in einem der Ansprüche 1 und 3 bis 6, mit einer aldehydischen Komponente b), definiert wie in einem der Ansprüche 8 bis 10, in Gegenwart eines Copolymers c) wie in einem der Ansprüche 1 und 11 bis 14 definiert, gegebenenfalls in Anwesenheit eines Kernmaterials, zusammengebracht und zur Reaktion gebracht werden, und
b) später durch Temperaturerhöhung die Aushärtung der Kapseln erfolgt, wobei auf die Verwendung von Formaldehyd verzichtet wird.

## Claims

1. Microcapsules whose capsule walls comprise a resin which is obtainable by reacting
a) at least one compound selected from the group of
a1) amines and
a2) aromatic or heteroaromatic compounds which are unsubstituted or substituted by one or more substituents from group C₁-C₂₀ alkyl, OH, OR, COOH, SH, SR, NHCOR, OCOR, halogen, aromatic, where R represents a C₁-C₁₀ alkyl group,
with
b) at least one aldehydic component which has at least two C atoms per molecule, in the presence of
c) at least one copolymer which comprises units of 2-acrylamido-2-methylpropanesulfonic acid or its salts (AMPS) and/or 2-acrylamido-2-methylpropanephosphonic acid or its salts (AMPP) and units of one or more (meth)acrylates,
wherein, the use of formaldehyde is excluded.

2. Microcapsules according to claim 1, wherein one or more compounds from the group of ureas, melamines and benzoguanamines are selected as component a).

3. Microcapsules according to claim 1, wherein one or more aromatic or heteroaromatic alcohols and/or one or more aromatic or heteroaromatic carboxylic acids are selected as component a).

4. Microcapsules according to claim 1 or 3, wherein an aromatic alcohol or ether thereof or derivative thereof is selected as component a).

5. Microcapsules according to any one of claims 3 or 4, wherein the at least one aromatic alcohol is selected from the phenols with two or more hydroxyl groups, preferably from pyrocatechol, resorcinol, hydroquinone, 1,4-naphtho-hydroquinone, phloroglucinol, pyrogallol, hydroxyhydroquinone, further preferably resorcinol and phloroglucinol.

6. Microcapsules according to any one of claims 3 to 5, wherein the alcohols are present in the form of their salts, ethers or esters and the carboxylic acids are present in the form of their salts and esters.

7. Microcapsules according to claim 1, wherein the component a) comprises a mixture of a1) one or more amines, defined as in any of claims 1 or 2, and a2) one or more aromatic or heteroaromatic compounds, defined as in any of claims 1 and 3 to 6.

8. Microcapsules according to any of the preceding claims, wherein the aldehydic component b) is selected from aliphatic and aromatic aldehydes.

9. Microcapsules according to any of the preceding claims, wherein the aldehydic component is selected from valeraldehyde, caproaldehyde, caprylaldehyde, decanal, succinaldehyde, cyclohexancarbaldehyde, cyclopentanecarbaldehyde, 2-methyl-1-propanal, 2-methylpropionaldehyde, acetaldehyde, acrolein, aldosterone, antimycin A, 8'-apo-β-caroten-8'-al, benzaldehyde, butanal, chloral, citral, citronellal, crotonaldehyde, dimethylaminobenzaldehyde, folinic acid, fosmidomycin, furfural, glutaraldehyde, glyceraldehyde, glycolaldehyde, glyoxal, glyoxylic acid, heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechualdehyde, retinal, salicylaldehyde, secologanin, streptomycin, strophantidin, tylosin, vanillin, and cinnamaldehyde.

10. Microcapsules according to any of the preceding claims, wherein the aldehydic component is selected from glutaraldehyde and/or succinaldehyde.

11. Microcapsules according to in any of the preceding claims, wherein the copolymer is a bipolymer or a terpolymer.

12. Microcapsules according to any of the preceding claims, wherein the copolymer c) is constructed from units of AMPS or AMPP, preferably AMPS, and one or more (meth)acrylates.

13. Microcapsules according to claim 1 or 12, wherein in copolymer c) the (meth)acrylates are selected from the group
a) 4-hydroxybutyl acrylate 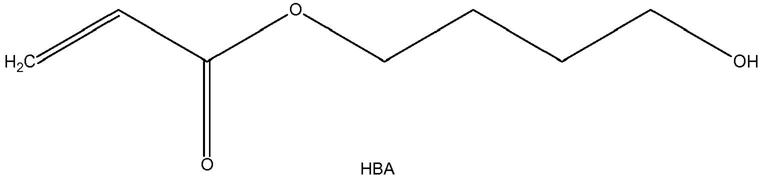
b) 2-hydroxypropyl methacrylate 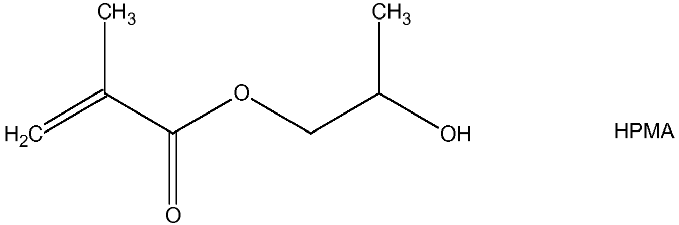
c) ammonium sulfatoethyl methacrylate 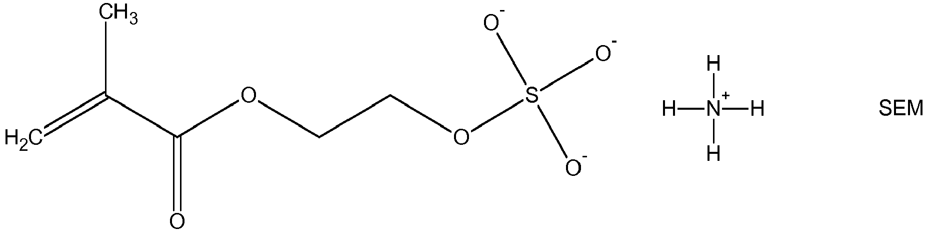
d) pentapropylene glycol methacrylate 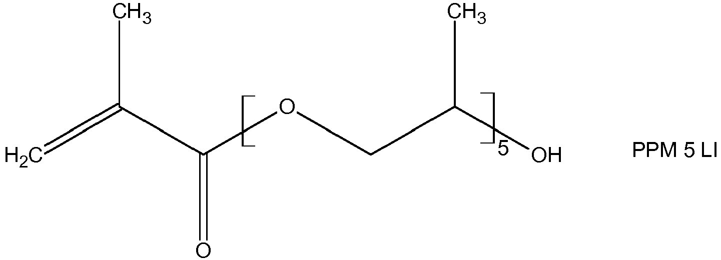
e) acrylic acid 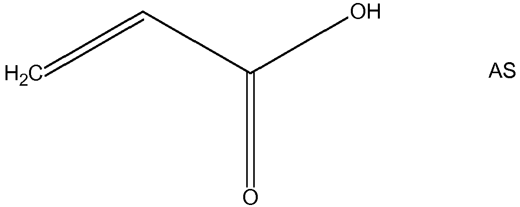
f) hexaethylene glycol methacrylate 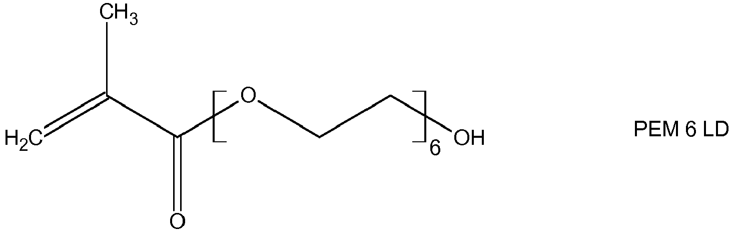
g) hexapropylene glycol acrylate 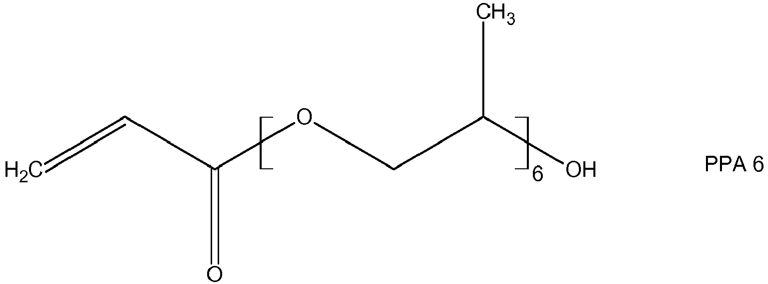
h) hexaethylene glycol acrylate 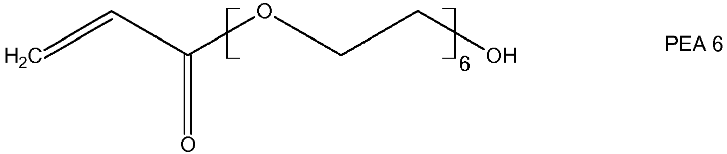
i) hydroxyethyl methacrylate 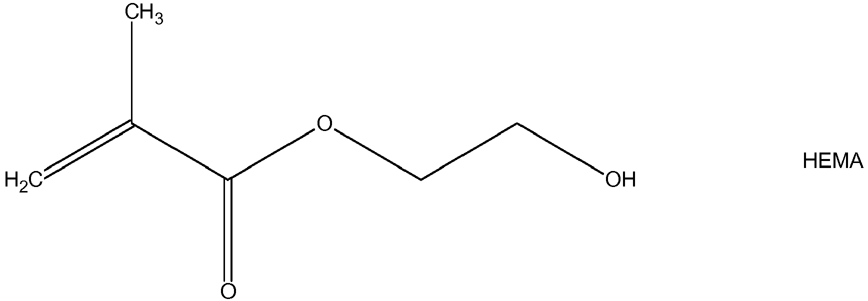
j) polyalkylene glycol methacrylate (CAS No. 589-75,9) 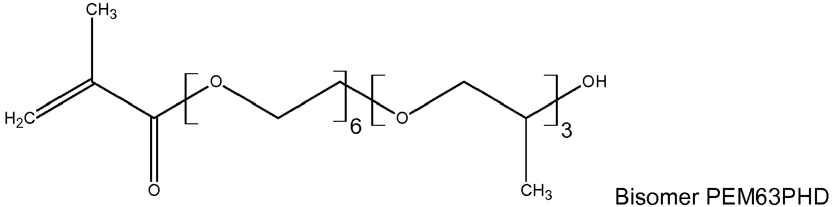
k) methoxy-polyethylene glycol methacrylate 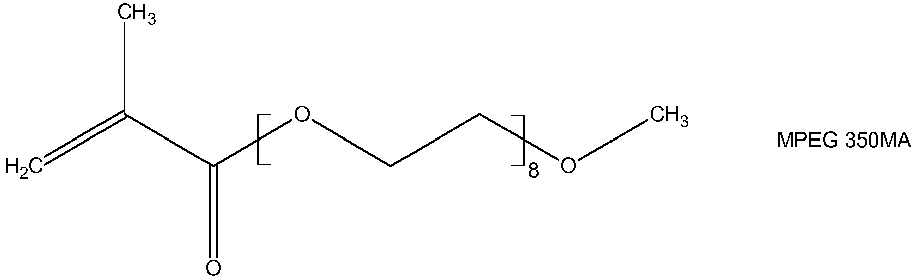
l) 2-propylheptyl acrylate (2-PHA) 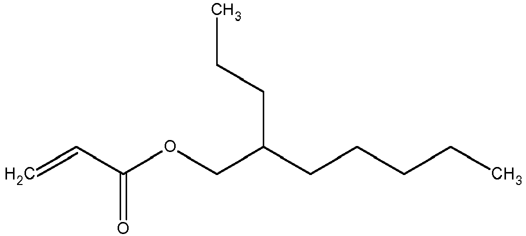
m) 1,3-butanediol dimethacrylate (BDDMA) 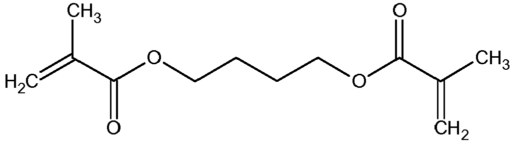
n) triethylene glycol dimethacrylate (TEGDMA) 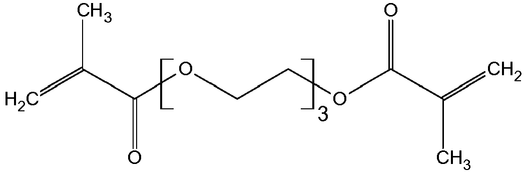
o) hydroxyethyl acrylate (HEA) 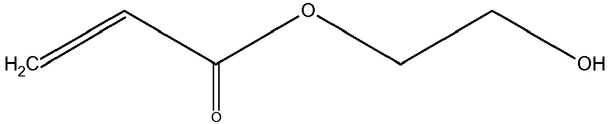
p) 2-hydroxypropyl acrylate (HPA) 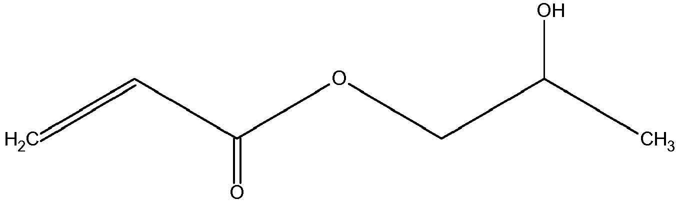
q) ethylene glycol dimethacrylate (EGDMA) 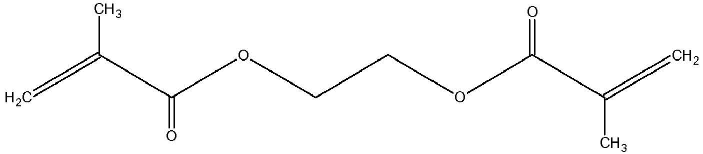
r) glycidyl methacrylate (GMA) 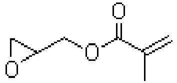
s) allyl methacrylate (ALMA) 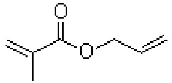
t) bisomer PEM 3 (polyethylene glycol methacrylate) 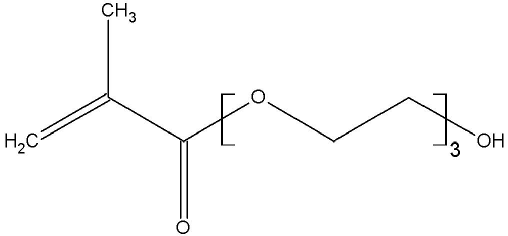
u) bisomer IDMA (Isodecyl methacrylate) 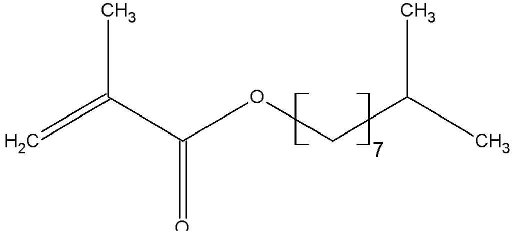
v) bisomer C13MA (isotridecyl methacrylate) 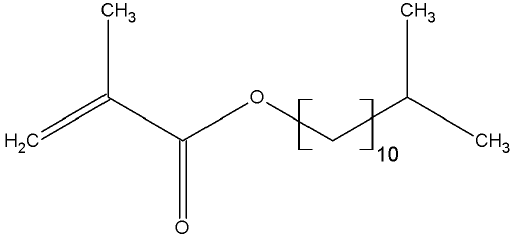
w) 3-sulfopropyl acrylate
x) N-(2-acryloyloxyethyl)-N,N-dimethyl-N-(3-sulfopropyl)ammonium betaine 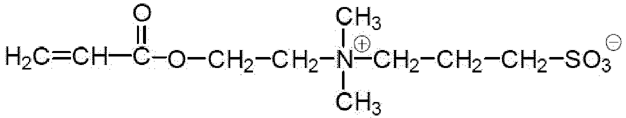
y) N-(2-methacryloyloxyethyl)-N,N-dimethyl-N-(3-sulfopropyl)ammonium betaine 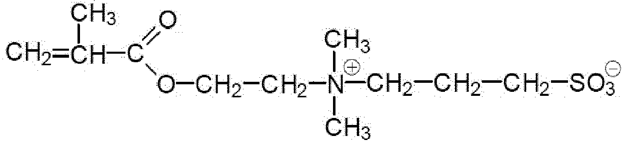
z) 3-sulfopropyl methacrylate,
z1) N-(3-methacrylamidopropyl)-N,N-dimethyl-N-(3-sulfopropyl)ammonium betaine

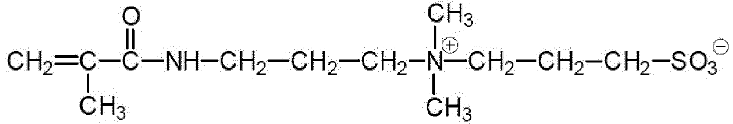

14. Microcapsules according to any of the preceding claims, wherein the copolymer c) comprises one or more further monomers selected from the group of vinyl compounds, unsaturated di- or polycarboxylic acids, and the salts of amyl compounds or allyl compounds.

15. Microcapsules according to any of the preceding claims, wherein the molar ratio of the at least one component a) to the at least one aldehydic component b) is between 1:1 and 1:5, preferably between 1:1 and 1:3.

16. Microcapsules according to any of the preceding claims, wherein the capsule surface is aftertreated with a nitrogen-containing agent, an oxygen-containing agent or a silicon-containing agent, preferably with melamine, with a silica gel, or aromatic alcohol a).

17. A method for manufacturing microcapsules according to one or more of claims 1 to 16, wherein
a) one or more amines a1), defined as in any of claims 1 to 2, and/or one or more aromatic or heteroaromatic compounds a2), defined as in any of claims 1 and 3 to 6, with an aldehydic component b), defined as in any of claims 8 to 10, in the presence of a copolymer c) as defined in any of claims 1 and 11 to 14, if applicable in the presence of a core material, are combined and induced to react, and
b) hardening of the capsules occurs later by temperature increase, whereby the use of formaldehyde is excluded.

## Revendications

1. Microcapsules, dont les parois de capsule comprennent une résine qui peut être obtenue par réaction
a) d'au moins un composé sélectionné parmi le groupe des
a1) amines et
a2) composés aromatiques ou hétéroaromatiques non substitués ou substitué avec un ou plusieurs substituant parmi le groupe contenant alkyle en C₁-C₂₀, OH, OR, COOH, SH, SR, NHCOR, OCOR, halogène, aromatique, R représentant un groupe alkyle en C₁-C₁₀,
avec
b) au moins un composant aldéhydique qui présente au moins deux atomes de C par molécule, en présence
c) d'au moins un copolymère qui contient des motifs de l'acide 2-acrylamido-2-méthyl-propanesulfonique ou de ses sels (AMPS) et/ou de l'acide 2-acrylamido-2-méthyl-propanephosphonique ou de ses sels (AMPP) et des motifs d'un ou plusieurs (méth)acrylates, en renonçant à l'utilisation de formaldéhyde.

2. Microcapsules selon la revendication 1, dans lesquelles on choisit en tant que composant a) un ou plusieurs composés parmi le groupe des urées, des mélamines et des benzoguanamines.

3. Microcapsules selon la revendication 1, dans lesquelles on choisit en tant que composant a) un ou plusieurs alcools aromatiques ou hétéroaromatiques et/ou un ou plusieurs acides carboxyliques aromatiques ou hétéroaromatiques.

4. Microcapsules selon la revendication 1 ou 3, dans lesquelles on choisit en tant que composant a) un alcool aromatique ou un éther de celui-ci ou un dérivé de celui-ci.

5. Microcapsules selon l'une quelconque des revendications 3 ou 4, dans lesquelles l'au moins un alcool aromatique est choisi parmi les phénols ayant deux ou plus de deux groupes hydroxyle, de préférence choisis parmi le pyrocatéchol, la résorcine, l'hydroquinone et la 1,4-naphtohydroquinone, la phloroglucine, le pyrogallol, l'hydroxyhydroquinone, et plus préférablement la résorcine et la phloroglucine.

6. Microcapsules selon l'une quelconque des revendications 3 à 5, dans lesquelles les alcools se présentent sous la forme de leurs sels, de leurs éthers ou de leurs esters et les acides carboxyliques se présentent sous la forme de leurs sels ou de leurs esters.

7. Microcapsules selon la revendication 1, dans lesquelles le composant a) contient un mélange de a1) une ou plusieurs amines, définies comme dans l'une des revendications 1 ou 2, et a2) un ou plusieurs composés aromatiques ou hétéroaromatiques, définis comme dans l'une des revendications 1 et 3 à 6.

8. Microcapsules selon l'une quelconque des revendications précédentes, dans lesquelles le composant aldéhydique b) est choisi parmi les aldéhydes aliphatiques et aromatiques.

9. Microcapsules selon l'une quelconque des revendications précédentes, dans lesquelles le composant aldéhydique est choisi parmi le valéraldéhyde, le capronaldéhyde, le carpylaldéhyde, le décanal, le succindialdéhyde, le cyclohexancarbaldéhyde, le cyclopentancarbaldéhyde, le 2-méthyl-1-propanal, le 2-méthylpropionaldéhyde, l'acétaldéhyde, l'acroléine, l'aldostérone, l'atinmycine A, le 8'-apo-β-carotén-8'-al, le benzaldéhyde, le butanal, le chloral, le citral, le citronellal, le crotonaldéhyde, le diméthylaminobenzaldéhyde, l'acide folique, la fosmidomycine, le furfural, le glutaraldéhyde, le glycérinaldéhyde, le glycolaldéhyde, le glyoxal, l'acide glyoxylique, l'heptanal, le 2-hydroxybenzaldéhyde, le 3-hydroxybutanal, l'hydroxyméthylfurfural, le 4-hydroxynonénal, l'isobutanal, l'isobutyraldéhyde, la méthacroléine, le 2-méthylundécanal, l'acide mucochlorique, le N-méthylformamide, le 2-nitrobenzaldéhyde, le nonanal, l'octanal, l'oléocanthal, l'orlistat, le pentanal, le phényléthanal, la phycocyanine, le pipéronal, le propanal, le propénal, le protocatéchualdéhyde, le rétinal, le salicylaldéhyde, la sécologanine, la streptomycine, la strophanthidine, la tylosine, la vanilline, l'aldéhyde cinnamique.

10. Microcapsules selon l'une quelconque des revendications précédentes, dans lesquelles le composant aldéhydique est choisi parmi le glutardialdéhyde et/ou le succindialdéhyde.

11. Microcapsules selon l'une quelconque des revendications précédentes, dans lesquelles le copolymère est un bipolymère ou un terpolymère.

12. Microcapsules selon l'une quelconque des revendications précédentes, dans lesquelles le copolymère c) est formé de motifs AMPS ou AMPP, de préférence AMPS, et d'un ou plusieurs (méth)acrylates.

13. Microcapsules selon la revendication 1 ou 12, dans lesquelles, dans le copolymère c), les (méth)acrylates sont choisis parmi le groupe comprenant
a) le 4-hydroxybutylacrylate 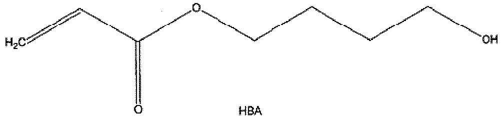
b) le méthacrylate de 2-hydroxypropyle 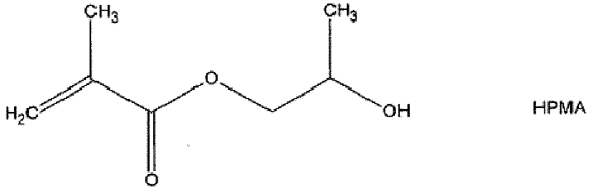
c) le sulfatoéthylméthacrylate d'ammonium 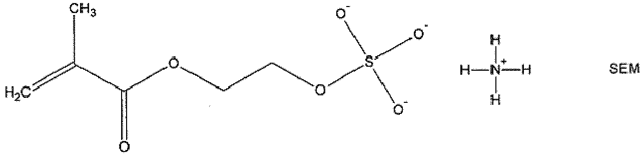
d) le méthacrylate de pentapropylèneglycol 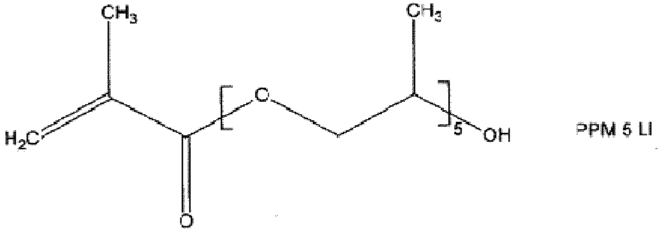
e) l'acide acrylique 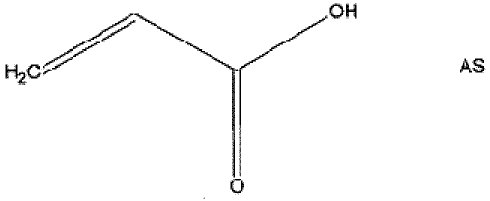
f) le méthacrylate d'hexaéthylèneglycol 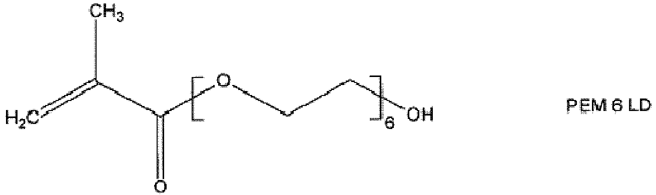
g) l'acrylate d'hexapropylèneglycol 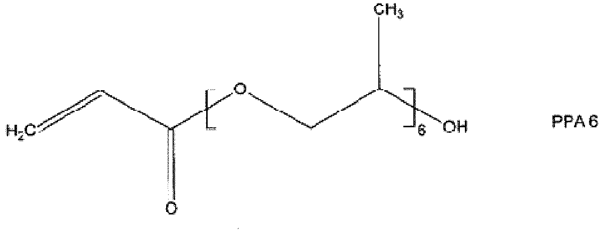
h) l'acrylate d'hexaéthylèneglycol 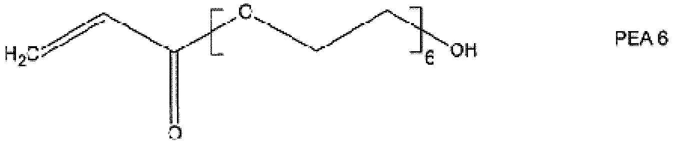
i) le méthacrylate d'hydroxyéthyle 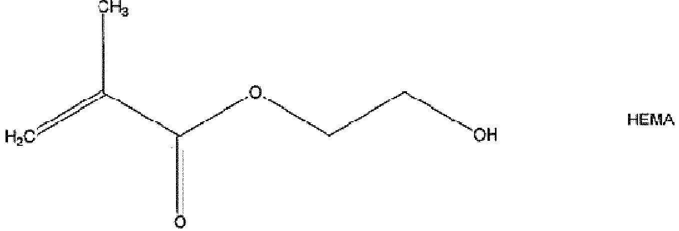
j) le méthacrylate de polyalkylèneglycol (n° CAS 589-75-9) 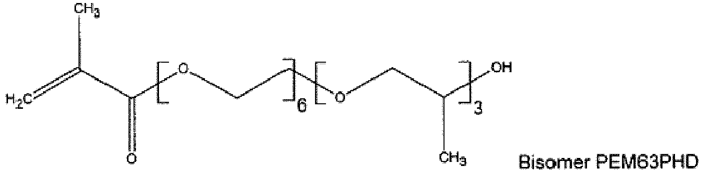
k) le méthacrylate de méthoxy-polyéthylèneglycol 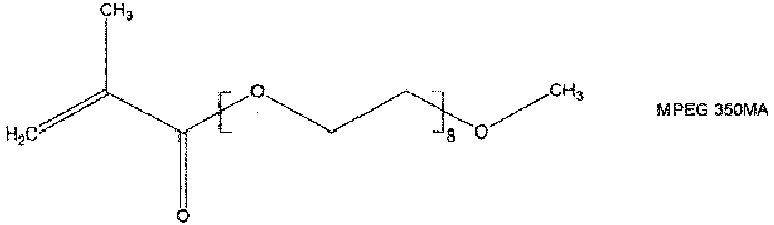
l) l'acrylate de 2-propylheptyle (2-PHA) 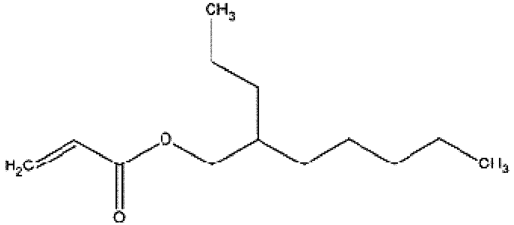
m) le diméthylacrylate de 1,3-butanediol (BDDMA) 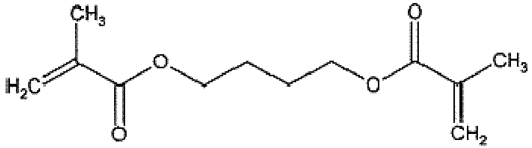
n) le diméthacrylate de triéthylèneglycol (TEGDMA) 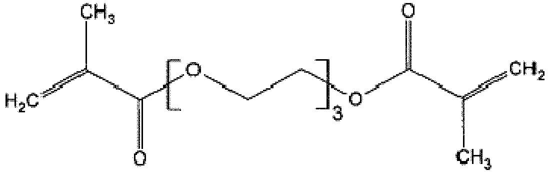
o) l'acrylate d'hydroxyéthyle (HEA) 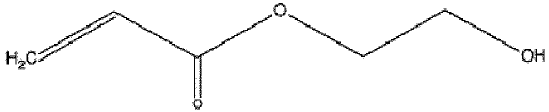
p) l'acrylate de 2-hydroxypropyle (HPA) 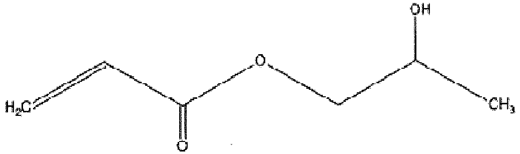
q) le diméthacrylate d'éthylèneglycol (EGDMA) 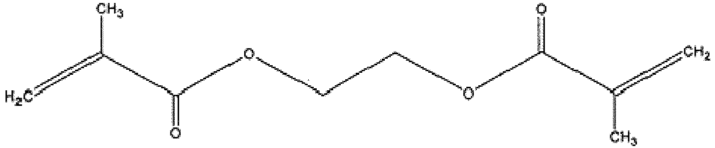
r) le méthacrylate de glycidyle (GMA) 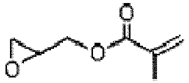
s) le méthacrylate d'allyle (ALMA) 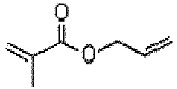
t) le bisomer PEM 3 (méthacrylate de polyéthylèneglycol) 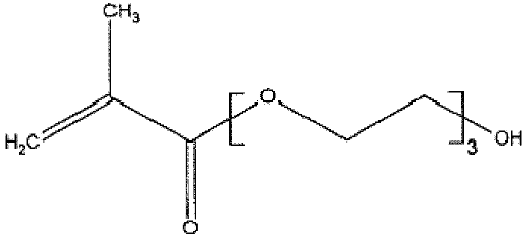
u) le bisomer IDMA (méthacrylate d'isodécyle) 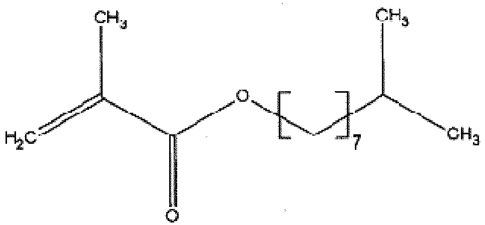
v) le bisomer C13MA (méthyacrylate d'isotridécyle) 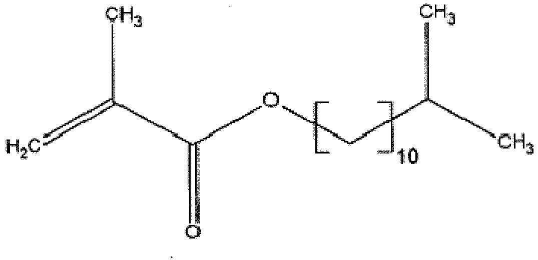
w) l'acrylate de 2-sulfopropyle
x) la N-(2-acryloyloxyéthyl)-N,N-diméthyl-N-(3-sulfopropyl)ammoniumbétaine 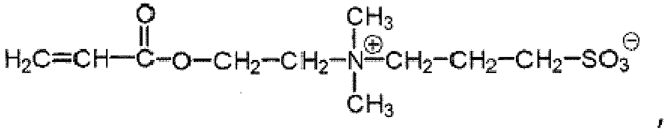
y) la N-(2-méthacryloyloxyéthyl)-N,N-diméthyl-N-(3-sulfopropyl)ammoniumbétaine 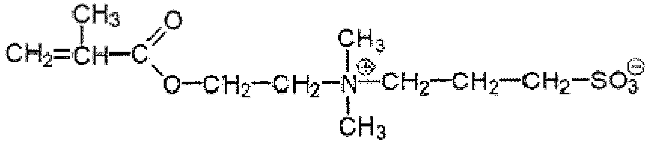
z) le méthacrylate de 3-sulfopropyle
z1) la N-(3-méthacrylamidopropyl)-N,N-diméthyl-N-(3-sulfopropyl)ammoniumbétaine

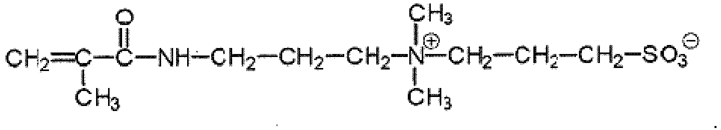

14. Microcapsules selon l'une quelconque des revendications précédentes, dans lesquelles le copolymère c) contient un ou plusieurs autres monomères choisis parmi le groupe des composés vinyliques, des acides di- ou polycarboxyliques insaturés et des sels de composés amyle ou allyle.

15. Microcapsules selon l'une quelconque des revendications précédentes, dans lesquelles le rapport molaire de l'au moins un composant a) à l'au moins un composant aldéhydique b) est compris entre 1:1 et 1:5, de préférence entre 1:1 et 1:3.

16. Microcapsules selon l'une quelconque des revendications précédentes, dans lesquelles la surface des capsules est post-traitée avec un agent contenant de l'azote, contenant de l'oxygène ou contenant du silicium, de préférence avec de la mélamine, un gel de silice ou l'alcool aromatique a) .

17. Procédé de préparation de microcapsules selon l'une quelconque ou plusieurs des revendications 1 à 16, dans lequel
a) une ou plusieurs amines a1), définies comme dans l'une des revendications 1 ou 2, et/ou un ou plusieurs composés aromatiques ou hétéroaromatiques a2), définis comme dans l'une des revendications 1 et 3 à 6, sont réunis et mis à réagir avec un composant aldéhyde b) défini comme dans l'une des revendications 8 à 10, en présence d'un copolymère c) défini comme dans l'une des revendications 1 et 11 à 14, éventuellement en présence d'un matériau de coeur, et
b) le durcissement des capsules s'effectue ultérieurement par augmentation de la température, en renonçant à l'utilisation de formaldéhyde.
